# EUROPEAN PATENT APPLICATION

(11) **EP 3 141 892 A1**
(43) Date of publication of application: **15.03.2017**
(21) Application number: 16801943.8
(22) Date of filing: 24.03.2016
(51) Int. Cl.: G01N 27/404, A61B 1/12, A61L 2/18, G01N 27/26, G01N 27/333, G01N 27/416

(54) **ENDOSCOPE REPROCESSOR**

(30) Priority: 05.06.2015 JP 2015114993
(71) Applicant: Olympus Corporation, Tokyo 192-8507 (JP)
(72) Inventor: AKAHORI, Hiromasa, Tokyo 192-8507 (JP)
(74) Representative: Gunzelmann, Rainer
(86) International application number: PCT/JP2016/059496
(87) International publication number: WO 2016/194449

(57) **Abstract**

An endoscope reprocessor includes a concentration meter including a permeation membrane, a medicinal-solution storing section including a holding section configured to hold the concentration meter such that the permeation membrane is disposed at a predetermined water level, the medicinal-solution storing section storing the medicinal solution to a water level higher than the predetermined water level, a liquid discharge section configured to discharge the medicinal solution from the medicinal-solution storing section, a liquid supply section configured to supply liquid to the permeation membrane of the concentration meter, and a control section connected to the liquid discharge section and the liquid supply section, the control section driving the liquid supply section to wet the permeation membrane when the medicinal solution in the medicinal-solution storing section is lower than the predetermined water level.

## Description

### Technical Field

The present invention relates to an endoscope reprocessor that applies reprocessing, in which a medicinal solution is used, to an endoscope.

### Background Art

An endoscope used in a medical field is subjected to reprocessing such as cleaning treatment and disinfecting treatment after use. An endoscope reprocessor that automatically performs the reprocessing of the endoscope is known. The endoscope reprocessor includes a medicinal solution tank that stores a medicinal solution used for the reprocessing. In order to determine whether the medicinal solution used for the reprocessing is usable, a concentration meter for measuring concentration of the medicinal solution disclosed in, for example, Japanese Patent Application Laid-Open Publication No. 2006-126046 is sometimes used.

When a measurement surface that comes into contact with a medicinal solution during concentration measurement is in a dried state, a concentration meter needs a longer standby time period until a correct measurement result can be obtained after the measurement surface comes into contact with the medicinal solution compared with when the measurement surface is in a wet state. In order to reduce the standby time period, in the concentration meter disclosed in Japanese Patent Application Laid-Open Publication No. 2006-126046, moisture retention of the measurement surface is performed by pressing the measurement surface against a sponge-like porous material wet by preservation liquid.

Depending on a structure of a concentration meter, a measurement surface sometimes is not instantaneously brought into a measurable wet state simply by pressing the measurement surface against the sponge-like porous material wet by the preservation liquid as in the concentration meter disclosed in Japanese Patent Application Laid-Open Publication No. 2006-126046. That is, it is likely that a standby time period occurs before concentration measurement of the medicinal solution by the concentration meter is started.

The present invention solves the problems explained above, and an object of the present invention is to provide an endoscope reprocessor that keeps a measurement surface of a concentration meter in a sufficient wet state.

### Disclosure of Invention

### Means for Solving the Problem

An endoscope reprocessor according to an aspect of the present invention includes: a concentration meter including a housing, an electrode housed in the housing, a permeation membrane held by the housing, and internal liquid encapsulated in the housing, the concentration meter measuring concentration of a medicinal solution; a medicinal-solution storing section including a holding section configured to hold the concentration meter such that the permeation membrane is disposed at a predetermined water level, the medicinal-solution storing section storing the medicinal solution to a water level higher than the predetermined water level; a liquid discharge section configured to discharge the medicinal solution from the medicinal-solution storing section; a liquid supply section configured to supply liquid to the permeation membrane of the concentration meter; and a control section connected to the liquid discharge section and the liquid supply section, the control section driving the liquid supply section to wet the permeation membrane when the medicinal solution in the medicinal-solution storing section is lower than the predetermined water level.

### Brief Description of the Drawings

Fig. 1 is a diagram showing a schematic configuration of an endoscope reprocessor of a first embodiment;
Fig 2 is a partial sectional view of a medicinal-solution storing section and a concentration meter of the first embodiment;
Fig. 3 is a flowchart showing a main routine of the endoscope reprocessor of the first embodiment;
Fig. 4 is a flowchart of a power saving mode of the endoscope reprocessor of the first embodiment;
Fig. 5 is a flowchart of moisture retaining operation of the endoscope reprocessor of the first embodiment;
Fig. 6 is a diagram showing a modification of a liquid supply section of the first embodiment;
Fig. 7 is a diagram showing a configuration of a detecting section disposed in a medicinal-solution storing section of a second embodiment;
Fig. 8 is a flowchart of moisture retaining operation of an endoscope reprocessor of the second embodiment;
Fig. 9 is a diagram showing a first modification of a detecting section of the second embodiment;
Fig. 10 is a diagram showing a second modification of the detecting section of the second embodiment;
Fig. 11 is a diagram showing a third modification of the detecting section of the second embodiment;
Fig. 12 is a diagram showing a schematic configuration of an endoscope reprocessor of a third embodiment;
Fig. 13 is a diagram showing a schematic configuration of an endoscope reprocessor of a fourth embodiment;
Fig. 14 is a diagram showing a modification of the endoscope reprocessor of the fourth embodiment;
Fig. 15 is a diagram showing a schematic configuration of an endoscope reprocessor of a fifth embodiment;
Fig. 16 is a diagram showing a schematic configuration of an endoscope reprocessor of a sixth embodiment;
Fig. 17 is a diagram showing a schematic configuration of an endoscope reprocessor of a seventh embodiment;
Fig. 18 is a flowchart showing a main routine of the endoscope reprocessor of the seventh embodiment;
Fig. 19 is a flowchart of a power saving mode of the endoscope reprocessor of the seventh embodiment; and
Fig. 20 is a flowchart of a moisture retaining operation of the endoscope reprocessor of the seventh embodiment.

### Best Mode for Carrying Out the Invention

Preferred modes of the present invention are explained below with reference to the drawings. Note that, in the respective figures used for the following explanation, in order to show respective components in recognizable sizes on the drawings, scales are varied for each of the components. The present invention is not limited to only numbers of the components, shapes of the components, ratios of sizes of the components, and relative positional relations among the respective components described in the figures.

### (First Embodiment)

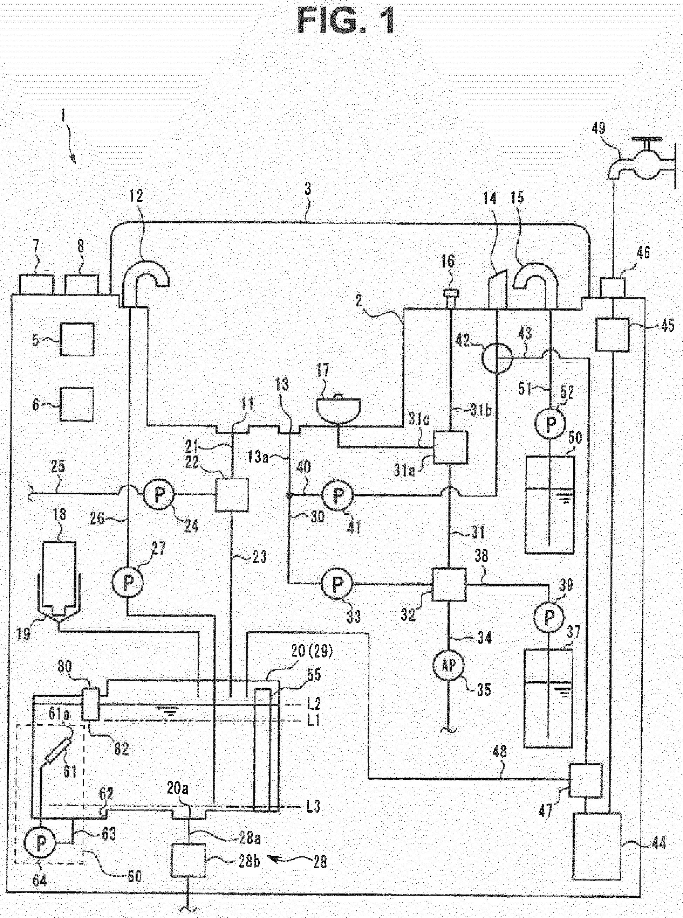

An example of an embodiment of the present invention is explained below. An endoscope reprocessor 1 shown in Fig. 1 is an apparatus that applies reprocessing to an endoscope. The reprocessing referred to herein is not particularly limited and may be any one of rinsing treatment by water, cleaning treatment for removing strains of organic matters and the like, disinfecting treatment for disabling predetermined microorganisms, sterilizing treatment for eliminating or killing all microorganisms, and a combination of the foregoing.

Note that, in the following explanation, upward indicates a position further away from a ground with respect to a comparison target and downward indicates a position closer to the ground with respect to the comparison target. High and low in the following explanation indicate a height relation along a gravity direction.

The endoscope reprocessor 1 includes a control section 5, a power supply section 6, a treatment tank 2, a medicinal-solution storing section 20, a concentration meter 80, a liquid discharge section 28, and a liquid supply section 60.

The control section 5 can include an arithmetic unit (a CPU), a storage device (a RAM), an auxiliary storage device, an input/output device, a power control device and the like. The control section 5 includes a configuration for controlling, based on a predetermined program, operations of respective parts configuring the endoscope reprocessor 1. Operations of the respective components included in the endoscope reprocessor 1 in the following explanation are controlled by the control section 5 even when not particularly described.

The power supply section 6 supplies electric power to the respective parts of the endoscope reprocessor 1. The power supply section 6 distributes electric power obtained from an outside such as a commercial power supply to the respective parts. Note that the power supply section 6 may include a power generating device or a battery.

The treatment tank 2 is formed in a concave shape including an opening section and is capable of storing liquid inside. A not-shown endoscope can be disposed in the treatment tank 2. In the present embodiment, as an example, a lid 3 for opening and closing the opening section of the treatment tank 2 is provided in an upper part of the treatment tank 2. When reprocessing is applied to the endoscope in the treatment tank 2, the opening section of the treatment tank 2 is closed by the lid 3.

In the treatment tank 2, a medicinal solution nozzle 12, a liquid discharge port 11, a circulation port 13, a circulation nozzle 14, a cleaning liquid nozzle 15, an endoscope connecting section 16, and an accessory case 17 are provided.

The medicinal solution nozzle 12 is an opening section that communicates with the medicinal-solution storing section 20 via a medicinal solution conduit 26. The medicinal-solution storing section 20 stores a medicinal solution. A type of the medicinal solution stored by the medicinal-solution storing section 20 is not particularly limited. However, in the present embodiment, as an example, the medicinal solution is disinfectant liquid such as acetic peracid used for the disinfecting treatment. However, the present invention is not limited to this. As the medicinal solution, cleaning liquid used for the cleaning treatment, a high-volatile solution used for drying, and the like can be selected as appropriate according to purposes.

In the present embodiment, as an example, the medicinal solution is obtained by diluting, with water, at a predetermined ratio, a stock solution of the medicinal solution supplied from a medicinal solution bottle 18. The medicinal-solution storing section 20 of the present embodiment communicates with a bottle connecting section 19 that introduces the stock solution of the medicinal solution supplied from the medicinal solution bottle 18 into the medicinal-solution storing section 20 and a dilution conduit 48 for introducing water for dilution into the medicinal-solution storing section 20. The medicinal solution bottle 18 is connected to the bottle connecting section 19, whereby the stock solution of the medicinal solution is introduced into the medicinal-solution storing section 20. A configuration for introducing the water from the dilution conduit 48 into the medicinal-solution storing section 20 is explained below.

Note that the endoscope reprocessor 1 does not have to include the configuration for diluting the medicinal solution with the water or the like. When a plurality of kinds of stock solutions are mixed and used as the medicinal solution, the bottle connecting section 19 is connectable to a plurality of medicinal solution bottles 18.

In the present embodiment, as an example, the medicinal solution is usable when concentration is within a predetermined range in which the medicinal solution has medicinal effects. When the endoscope reprocessor 1 is not executing operation in which the medicinal solution is used, the medicinal-solution storing section 20 stores the entire medicinal solution in the endoscope reprocessor 1. That is, in the present embodiment, the medicinal-solution storing section 20 also functions as a medicinal-solution collecting section 29 that collects and stores the medicinal solution in the endoscope reprocessor 1. In the following explanation, when the medicinal-solution storing section 20 and the medicinal-solution collecting section 29 are not distinguished, the medicinal-solution storing section 20 and the medicinal-solution collecting section 29 are simply referred to as medicinal-solution storing section 20.

Note that the medicinal-solution storing section 20 may be provided separately from the medicinal-solution collecting section 29. When the medicinal-solution storing section 20 is a component different from the medicinal-solution collecting section 29, the capacity of the medicinal-solution storing section 20 may be smaller than the capacity of the medicinal-solution collecting section 29.

In the medicinal-solution storing section 20, the concentration meter 80, the liquid discharge section 28, and the liquid supply section 60 are also disposed. Detailed configurations of these components and the medicinal-solution storing section 20 are explained below.

A medicinal solution pump 27 is provided in the medicinal solution conduit 26. By operating the medicinal solution pump 27, the medicinal solution in the medicinal-solution storing section 20 is transferred into the treatment tank 2 through the medicinal solution conduit 26 and the medicinal solution nozzle 12.

The liquid discharge port 11 is an opening section provided in a lowest part in the treatment tank 2. The liquid discharge port 11 is connected to a discharge conduit 21. The discharge conduit 21 causes the liquid discharge port 11 and a switching valve 22 to communicate. A collection conduit 23 and a discarding conduit 25 are connected to the switching valve 22. The switching valve 22 can be switched to a state in which the discharge conduit 21 is closed, a state in which the discharge conduit 21 and the collection conduit 23 are caused to communicate, or a state in which the discharge conduit 21 and the discarding conduit 25 are caused to communicate.

The collection conduit 23 causes the medicinal-solution storing section 20 and the switching valve 22 to communicate. A discharge pump 24 is provided in the discarding conduit 25. The discarding conduit 25 is connected to a liquid discharge facility for receiving the liquid discharged from the endoscope reprocessor 1.

If the switching valve 22 is switched to a closed state, it is possible to store the liquid in the treatment tank 2. When the medicinal solution is stored in the treatment tank 2, if the switching valve 22 is switched to the state in which the discharge conduit 21 and the collection conduit 23 communicate, the medicinal solution is transferred from the treatment tank 2 to the medicinal-solution storing section 20. If the switching valve 22 is switched to the state in which the discharge conduit 21 and the discarding conduit 25 communicate and the operation of the discharge pump 24 is started, the liquid in the treatment tank 2 is delivered to the liquid discharge facility through the discarding conduit 25.

The circulation port 13 is an opening section provided near a bottom surface of the treatment tank 2. The circulation port 13 communicates with a circulation conduit 13a. The circulation conduit 13a branches to two conduits of an endoscope circulation conduit 30 and a treatment tank circulation conduit 40.

The endoscope circulation conduit 30 causes the circulation conduit 13a and a channel valve 32 explained below to communicate. A circulation pump 33 is provided in the endoscope circulation conduit 30. The circulation pump 33 operates to transfer fluid in the endoscope circulation conduit 30 toward the channel valve 32.

Besides the endoscope circulation conduit 30, an air intake conduit 34, an alcohol conduit 38, and a delivery conduit 31 are connected to the channel valve 32. The channel valve 32 selectively causes any one of the endoscope circulation conduit 30, the air intake conduit 34, and the alcohol conduit 38 to communicate with the delivery conduit 31.

One end portion of the air intake conduit 34 is opened to an atmosphere. The other end portion is connected to the channel valve 32. Note that, although not shown in the figure, a filter for filtering passing gas is provided at the one end portion of the air intake conduit 34. An air pump 35 is provided in the air intake conduit 34. The air pump 35 operates to thereby transfer the gas in the air intake conduit 34 toward the channel valve 32.

The alcohol conduit 38 causes an alcohol tank 37 that stores alcohol and the channel valve 32 to communicate. Examples of the alcohol stored in the alcohol tank 37 include ethanol. Alcohol concentration can be selected as appropriate. An alcohol pump 39 is provided in the alcohol conduit 38. The alcohol pump 39 operates to thereby transfer the alcohol in the alcohol tank 37 toward the channel valve 32.

When liquid is stored in the treatment tank 2, if the channel valve 32 is switched to a state in which the delivery conduit 31 and the endoscope circulation conduit 30 communicate and operation of the circulation pump 33 is started, the liquid in the treatment tank 2 is sent into the delivery conduit 31 through the circulation port 13, the circulation conduit 13a, and the endoscope circulation conduit 30.

If the channel valve 32 is switched to a state in which the delivery conduit 31 and the air intake conduit 34 communicate and operation of the air pump 35 is started, the air is sent into the delivery conduit 31. If the channel valve 32 is switched to a state in which the delivery conduit 31 and the alcohol conduit 38 communicate and operation of the alcohol pump 39 is started, the alcohol in the alcohol tank 37 is sent into the delivery conduit 31.

The delivery conduit 31 branches to an endoscope connection conduit 31b and a case connection conduit 31c. The endoscope connection conduit 31b is connected to the endoscope connecting section 16. The case connection conduit 31c is connected to the accessory case 17.

A channel switching section 31 a is provided in the delivery conduit 31. The channel switching section 31a is capable of switching to which of the endoscope connection conduit 31b and the case connection conduit 31c the fluid sent into the delivery conduit 31 from the channel valve 32 is fed. Note that pressure on the endoscope connection conduit 31b side may be controlled to be constant during the switching.

The endoscope connecting section 16 is connected to a pipe sleeve provided in the endoscope via a not-shown endoscope tube. The accessory case 17 is a basket-like member that houses not-shown accessories of the endoscope. Therefore, the fluid sent into the delivery conduit 31 from the channel valve 32 is introduced into the pipe sleeve of the endoscope or into the accessory case 17.

The treatment tank circulation conduit 40 causes the circulation conduit 13a and the circulation nozzle 14 to communicate. The circulation nozzle 14 is an opening section provided in the treatment tank 2. A circulation pump 41 is provided in the treatment tank circulation conduit 40.

A three-way valve 42 is provided between the circulation pump 41 of the treatment tank circulation conduit 40 and the circulation nozzle 14. A water supply conduit 43 is connected to the three-way valve 42. The three-way valve 42 can be switched to a state in which the circulation nozzle 14 and the treatment tank circulation conduit 40 are caused to communicate or a state in which the circulation nozzle 14 and the water supply conduit 43 are caused to communicate.

The water supply conduit 43 causes the three-way valve 42 and a water-supply-source connecting section 46 to communicate. In the water supply conduit 43, a water introducing valve 45 for opening and closing the water supply conduit 43 and a water filter 44 for filtering water are provided. The water-supply-source connecting section 46 is connected to, via a hose or the like, a water supply source 49 such as waterworks that delivers water.

A dilution valve 47 is provided in a section between the water filter 44 and the three-way valve 42 of the water supply conduit 43. The dilution conduit 48 that causes the dilution valve 47 and the medicinal-solution storing section 20 to communicate is connected to the dilution valve 47. The dilution valve 47 can be switched to a state in which the water filter 44 and the three-way valve 42 are caused to communicate or a state in which the water filter 44 and the dilution conduit 48 are caused to communicate.

When liquid is stored in the treatment tank 2, if the three-way valve 42 is switched to the state in which the circulation nozzle 14 and the treatment tank circulation conduit 40 are caused to communicate, the dilution valve 47 is switched to the state in which the water filter 44 and the three-way valve 42 are caused to communicate, and operation of the circulation pump 41 is started, the liquid in the treatment tank 2 is discharged from the circulation nozzle 14 through the circulation port 13, the circulation conduit 13a, and the treatment tank circulation conduit 40.

If the three-way valve 42 is switched to the state in which the circulation nozzle 14 and the water supply conduit 43 are caused to communicate, the dilution valve 47 is switched to the state in which the water filter 44 and the three-way valve 42 are caused to communicate, and the water introducing valve 45 is switched to the open state, the water supplied from the water supply source 49 is discharged from the circulation nozzle 14. The liquid discharged from the circulation nozzle 14 is introduced into the treatment tank 2.

If the dilution valve 47 is switched to the state in which the water filter 44 and the dilution conduit 48 are caused to communicate and the water introducing valve 45 is switched to the open state, the water supplied from the water supply source 49 is introduced into the medicinal-solution storing section 20.

The cleaning liquid nozzle 15 is an opening section that communicates with, via a cleaning liquid conduit 51, a cleaning liquid tank 50 that stores the cleaning liquid. The cleaning liquid is used for the cleaning treatment. A cleaning liquid pump 52 is provided in the cleaning liquid conduit 51. When the cleaning liquid pump 52 is operated, the cleaning liquid in the cleaning liquid tank 50 is transferred into the treatment tank 2.

The endoscope reprocessor 1 includes an operation section 7 and an output section 8 configuring a user interface that performs exchange of information with a user. The operation section 7 and the output section 8 are electrically connected to the control section 5.

The operation section 7 includes an operation member such as a push switch or a touch sensor. The output section 8 includes, for example, a display device that displays images and characters, a light emitting device that emits light, a speaker that emits sound, or a combination of the foregoing. Note that the operation section 7 and the output section 8 may be included in an electronic device that performs wireless communication with the control section 5.

A configuration of the medicinal-solution storing section 20 is explained. In the medicinal-solution storing section 20, the concentration meter 80, a water level sensor 55, the liquid discharge section 28, and the liquid supply section 60 are disposed.

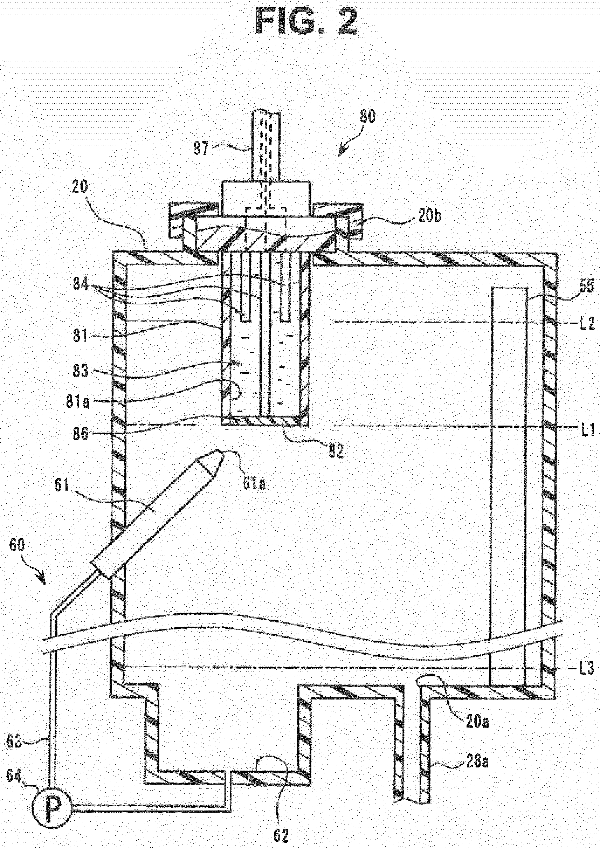

The concentration meter 80 measures concentration of a specific substance, which is a measurement target, in the medicinal solution in contact with a measurement surface 82. A partial sectional view of the medicinal-solution storing section 20 and the concentration meter 80 is shown in Fig. 2.

The concentration meter 80 may be included in the endoscope reprocessor 1 and electrically connected to the control section 5 or may operate alone without being included in the endoscope reprocessor 1. In the present embodiment, as an example, the concentration meter 80 is electrically connected to the control section 5. Information concerning a measurement result of concentration of the medicinal solution measured by the concentration meter 80 is inputted to the control section 5.

The measurement surface 82 is provided in a part of an outer surface of a housing 81. The housing 81 is a container-like member in which an opening section 81 a is provided. The opening section 81 a is sealed by a permeation membrane 86. Internal liquid 83 is encapsulated in an inside of the opening section 81a of the housing 81.

The measurement surface 82 is a surface on an opposite side of a region of the permeation membrane 86 in contact with the internal liquid 83. The permeation membrane 86 allows a substance, which is a measurement target, in the medicinal solution in contact with the measurement surface 82 to permeate into the internal liquid 83 side. That is, substance concentration in the internal liquid 83 changes according to substance concentration of the medicinal solution in contact with the measurement surface 82.

In the housing 81, a plurality of electrodes 84 are disposed spaced apart from one another in the internal liquid 83. The plurality of electrodes 84 are connected to a not-shown control device of the concentration meter 80 via an electric cable 87. Note that the control device of the housing 81 may be configured integrally with the housing 81. As explained above, in the present embodiment, the control device of the concentration meter 80 is included in the control section 5.

The concentration meter 80 measures a change in a potential difference that occurs among the plurality of electrodes 84 immersed in the internal liquid 83 or a change in a value of an electric current flowing between a pair of electrodes 84 and measures, based on a value of the measurement, concentration of the medicinal solution in contact with the measurement surface 82. Since a principle and a configuration of the concentration measurement in the concentration meter 80 are well known, detailed explanation of the principle and the configuration is omitted.

In the medicinal-solution storing section 20, a holding section 20b that holds the housing 81 of the concentration meter 80 is provided. The measurement surface 82 of the concentration meter 80 held by the holding section 20b is disposed at a predetermined first water level L1 in the medicinal-solution storing section 20. In the present embodiment, the measurement surface 82 of the concentration meter 80 held by the holding section 20b faces downward. The predetermined water level L1 is height of a liquid surface of the medicinal solution in the medicinal-solution storing section 20 in the case in which a predetermined volume of the medicinal solution is stored in the medicinal-solution storing section 20. The predetermined volume is smaller than a volume of the medicinal solution at least necessary for execution of the reprocessing by the endoscope reprocessor 1.

Height of the liquid surface of the medicinal solution in the medicinal-solution storing section 20 in the case in which the volume of the medicinal solution at least necessary for the execution of the reprocessing by the endoscope reprocessor 1 is stored in the medicinal-solution storing section 20 is represented as second water level L2. The second water level L2 is located above the first water level L1. That is, the medicinal-solution storing section 20 is capable of storing the medicinal solution to a water level higher than the first water level L1.

The water level sensor 55 detects height of the liquid surface of the medicinal solution stored in the medicinal-solution storing section 20. The water level sensor 55 is electrically connected to the control section 5 and outputs information concerning a detection result to the control section 5.

In the present embodiment, as an example, the water level sensor 55 at least detects whether the liquid surface in the medicinal-solution storing section 20 has reached the second water level L2 and whether the liquid surface of the medicinal solution has reached a third water level L3 explained below. The third water level L3 is located below the first water level L1. That is, the third water level L3 is located below the measurement surface 82 of the concentration meter 80. In the present embodiment, as an example, the third water level L3 is near a bottom surface of the medicinal-solution storing section 20.

Note that, when the stock solution of the medicinal solution supplied from the medicinal solution bottle 18 and the water supplied from the dilution conduit are mixed in the medicinal-solution storing section 20, the water level sensor 55 may be used to set a volume ratio of the stock solution and the water to a predetermined value.

The configuration of the water level sensor 55 is not particularly limited. The water level sensor 55 may be a so-called electrode-type water level sensor including, for example, a plurality of electrodes disposed spaced apart from one another, the electrode-type water level sensor detecting, based on presence or absence of electric conduction among the plurality of electrodes that changes according to whether the plurality of electrodes are sunk in liquid, whether the liquid surface has reached a predetermined water level. For example, the water level sensor 55 may be a so-called float-type water level sensor that detects, based on an operation state of a switch that opens and closes according to up and down movements of a float floating on the medicinal solution, whether the liquid surface of the medicinal solution has reached the predetermined water level.

The liquid discharge section 28 discharges liquid such as the medicinal solution or the water from the medicinal-solution storing section 20. The liquid discharge section 28 may be configured to discharge the liquid from the medicinal-solution storing section 20 with gravity or may be configured to forcibly discharge the liquid from the medicinal-solution storing section 20 with a pump.

In the present embodiment, as an example, the liquid discharge section 28 includes a drain conduit 28a that communicates with a liquid discharge port 20a provided on the bottom surface of the medicinal-solution storing section 20 or near the bottom surface and a drain valve 28b that opens and closes the drain conduit 28a. The drain valve 28b may be an electromagnetic on-off valve controlled to open and close by the control section 5 or may be a cock opened and closed by manual operation of the user.

Note that a route for discharging the liquid from the medicinal-solution storing section 20 is not limited to the drain conduit. For example, in the present embodiment, the user connects a not-shown collection container to the medicinal solution nozzle 12 and thereafter start operation of the medicinal solution pump 27, whereby it is also possible to discharge the liquid from the medicinal-solution storing section 20 into the collection container through the medicinal solution conduit 26 and the medicinal solution nozzle 12. In this case, the medicinal solution conduit 26 and the medicinal solution pump 27 are also included in the liquid discharge section 28.

In the present embodiment, as an example, the liquid discharge section 28 includes the medicinal solution conduit 26 and the medicinal solution pump 27. When an instruction for discharging the liquid from the medicinal-solution storing section 20 is inputted via the operation section 7, first, the endoscope reprocessor 1 of the present embodiment starts the operation of the medicinal solution pump 27 and, at a point in time when it is detected by the water level sensor 55 that the height of the liquid surface in the medicinal-solution storing section 20 is less than the third water level L3, stops the medicinal solution pump 27. In the operation, the liquid is discharged from the medicinal solution nozzle 12. The user can discharge the liquid remaining in the medicinal-solution storing section 20 at a point in time of the stop of the medicinal solution pump 27 through the drain conduit 28a by switching the drain valve 28b to an open state.

The liquid supply section 60 supplies the liquid to the permeation membrane 86 of the concentration meter 80 from an outer side of the housing 81. That is, the liquid supply section 60 supplies the liquid to the measurement surface 82 of the concentration meter 80.

As shown in Fig. 2, the liquid supply section 60 includes a nozzle 61 including an opening 61 a for discharging the liquid. In the present embodiment, the nozzle 61 is disposed such that the opening 61a faces the measurement surface 82. At least a part of the liquid discharged from the opening 61 a of the nozzle 61 is blown against the measurement surface 82 and wets the measurement surface 82. Note that the liquid supply section 60 may include a plurality of nozzles 61. The nozzle 61 may include a plurality of openings 61 a.

A configuration for discharging the liquid from the opening 61a of the nozzle 61 in the liquid supply section 60 is not particularly limited. The liquid supply section 60 may discharge, from the nozzle 61, liquid supplied from a device on an outside of the endoscope reprocessor 1 or may discharge, from the nozzle 61, liquid stored on an inside of the endoscope reprocessor 1 in advance.

Pressure applied to the liquid in order to discharge the liquid from the nozzle 61 may be generated by a device on the outside of the endoscope reprocessor 1 or may be generated by a device included in the inside of the endoscope reprocessor 1.

For example, the liquid supply section 60 may be connected to a device on the outside that discharges liquid of the water supply source 49 or the like at predetermined pressure and discharge the liquid from the opening 61 a of the nozzle 61 with pressure of the liquid. For example, the liquid supply section 60 may include a holding section that holds the liquid above the opening 61a and discharge the liquid from the opening 61a of the nozzle 61 with a water level difference between the holding section and the opening 61a. For example, the liquid supply section 60 may include a pump that delivers the liquid toward the nozzle 61.

A type of the liquid supplied to the measurement surface 82 by the liquid supply section 60 is not particularly limited. However, the liquid is desirably a medicinal solution or water, which is a measurement target of the concentration meter 80.

In the present embodiment, as an example, the liquid supply section 60 of the present embodiment includes a liquid holding section 62 that stores a predetermined amount of liquid, a supply conduit 63 that causes the liquid holding section 62 and the nozzle 61 to communicate, and a discharge pump 64 provided in the supply conduit 63.

The liquid holding section 62 of the present embodiment is a container having a concave shape opening upward below the second water level L2 in the medicinal-solution storing section 20. If the liquid such as the medicinal solution or the water is stored in the medicinal-solution storing section 20 up to an upper part the opening of the liquid holding section 62, the liquid holding section 62 is filled with the liquid. Thereafter, if the liquid in the medicinal-solution storing section 20 is discharged by the liquid discharge section 28, a predetermined amount of the liquid is stored in the liquid holding section 62.

In the present embodiment, as an example, the liquid holding section 62 is a concave section provided on the bottom surface of the medicinal-solution storing section 20 and opening upward. The liquid holding section 62 opens in a position different from the liquid discharge port 20a. Therefore, even after the liquid in the medicinal-solution storing section 20 is discharged from the liquid discharge port 20a by the liquid discharge section 28, the liquid is stored in the liquid holding section 62. Note that, although not shown in the figure, the endoscope reprocessor 1 includes a component such as a cock for discharging the liquid stored in the liquid holding section 62 to the outside of the apparatus.

The discharge pump 64 is electrically connected to the control section 5. Operation of the discharge pump 64 is controlled by the control section 5. The discharge pump 64 operates to thereby transfer liquid in the supply conduit 63 toward the nozzle 61. That is, the discharge pump 64 is operated, whereby the liquid stored in the liquid holding section 62 is discharged from the opening 61a of the nozzle 61.

At least a part of the liquid discharged from the opening of the nozzle 61 is blown against the measurement surface 82 of the concentration meter 80 and, after wetting the measurement surface 82, drops into the medicinal-solution storing section 20. In the present embodiment, since the liquid holding section 62 opens on the bottom surface of the medicinal-solution storing section 20. Therefore, the liquid discharged from the opening of the nozzle 61 returns into the liquid holding section 62 again. Note that the liquid discharged from the opening of the nozzle 61 does not have to return to the liquid holding section 62.

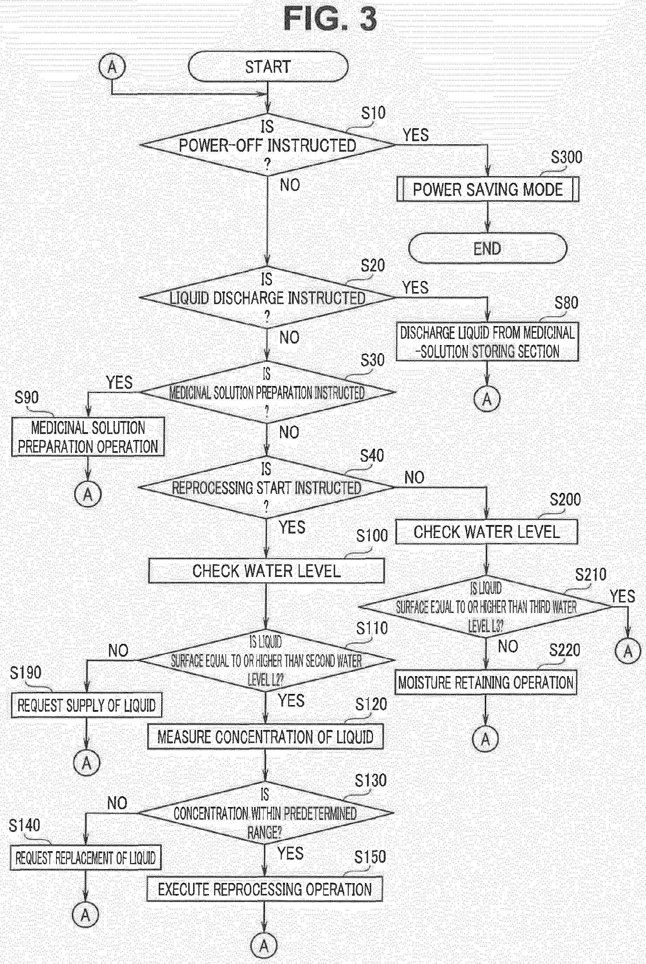

Operation of the endoscope reprocessor 1 is explained with reference to flowcharts shown in Fig. 3, Fig. 4, and Fig. 5. A flow shown in Fig. 3 is started, for example, when a power supply of the endoscope reprocessor 1 is brought into an ON state. Note that an input of an operation instruction to the endoscope reprocessor 1 from the user is performed via the operation section 7.

After the power supply of the endoscope reprocessor 1 is brought into the ON state, first, the endoscope reprocessor 1 executes initialization operation for the respective components. Then, as shown in steps S10 to S40, the endoscope reprocessor 1 stays on standby until an input of an instruction from the user is performed.

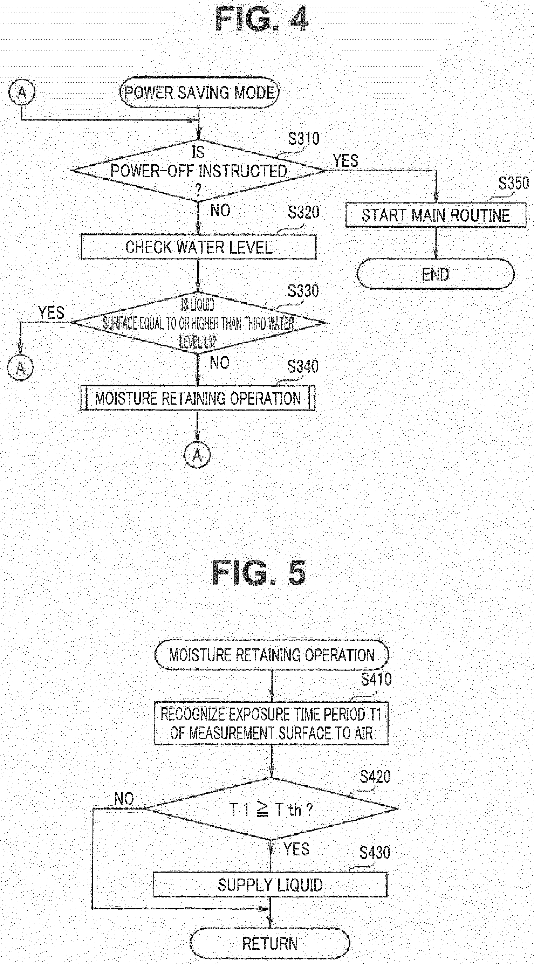

More specifically, in step S10, the endoscope reprocessor 1 determines whether an instruction for power-off is inputted by the user. When determining in step S10 that the instruction for power-off is inputted, the endoscope reprocessor 1 shifts to step S300 explained below, starts a power saving mode, which is a process executed during the power-off, shown in the flowchart of Fig. 4, and ends the flow shown in Fig. 3. In the power saving mode, the endoscope reprocessor 1 is in a state in which electric power is supplied to only components necessary for execution of the flowchart of Fig. 4 and supply of electric power to the other components is interrupted.

When determining in step S10 that the instruction for the power-off is not inputted, the endoscope reprocessor 1 shifts to step S20. In step S20, the endoscope reprocessor 1 determines whether an instruction for discharging the liquid such as the medicinal solution or the water in the medicinal-solution storing section 20 from the endoscope reprocessor 1 is inputted by the user. Operation for discharging the liquid from the endoscope reprocessor 1 is executed, for example, when the medicinal solution in the medicinal-solution storing section 20 is replaced or when the endoscope reprocessor 1 is not used for a relatively long time period. In the present embodiment, as an example, the user inputs the instruction for discharging the liquid in the medicinal-solution storing section 20 after connecting a not-shown collection container to the medicinal solution nozzle 12.

When determining in step S20 that the instruction for discharging the liquid in the medicinal-solution storing section 20 is inputted, the endoscope reprocessor 1 shifts to step S80 and executes a discharge process. In step S80, the endoscope reprocessor 1 starts operation of the medicinal solution pump 27 and stops the medicinal solution pump 27 at a point in time when it is detected by the water level sensor 55 that the height of the liquid surface in the medicinal-solution storing section 20 is less than the third water level L3. At this point in time, the liquid remains below the third water level L3 in the medicinal-solution storing section 20. When discharging the remaining liquid from the medicinal-solution storing section 20, the user switches the drain valve 28b to the open state.

Immediately after completion of step S80, the liquid is stored only in the liquid holding section 62 provided in the medicinal-solution storing section 20. Immediately after the completion of step S80, the measurement surface 82 of the concentration meter 80 is in contact with the air in the medicinal-solution storing section 20.

When determining in step S20 that the instruction for discharging the liquid in the medicinal-solution storing section 20 is not inputted, the endoscope reprocessor 1 shifts to step S30.

In step S30, the endoscope reprocessor 1 determines whether an instruction for preparing a medicinal solution in the medicinal-solution storing section 20 is inputted by the user. In the present embodiment, as an example, after connecting the medicinal solution bottle 18, in which a stock solution of an unused medicinal solution is stored, to the bottle connecting section 19, the user inputs the instruction for preparing a medicinal solution in the medicinal-solution storing section 20.

When determining in step S30 that the instruction for preparing a medicinal solution in the medicinal-solution storing section 20 is inputted, the endoscope reprocessor 1 shifts to step S90 and executes medicinal solution preparation operation. In the medicinal solution preparation operation in step S90, the endoscope reprocessor 1 switches the dilution valve 47 to the state in which the water filter 44 and the dilution conduit 48 are caused to communicate and switches the water introducing valve 45 to the open state to thereby introduce the water supplied from waterworks 59 into the medicinal-solution storing section 20. The water introduced into the medicinal-solution storing section 20 is mixed with the stock solution of the medicinal solution from the bottle connecting section 19. The endoscope reprocessor 1 stops the introduction of the water into the medicinal-solution storing section 20 at a point in time when it is detected by the water level sensor 55 that the height of the liquid surface in the medicinal-solution storing section 20 has reached a predetermined water level above the second water level L2.

Immediately after completion of step S90, a new medicinal solution obtained by mixing the stock solution of the unused medicinal solution and the water at a predetermined ratio is stored to the predetermined water level above the second water level L2 in the medicinal-solution storing section 20.

When determining in step S30 that the instruction for preparing the medicinal solution in the medicinal-solution storing section 20 is not inputted, the endoscope reprocessor 1 shifts to step S40.

In step S40, the endoscope reprocessor 1 determines whether an instruction for executing reprocessing for the endoscope is inputted by the user. When determining in step S40 that the instruction for executing the reprocessing is inputted, the endoscope reprocessor 1 shifts to step S100.

In step S100, the endoscope reprocessor 1 checks, with the water level sensor 55, whether the height of the liquid surface in the medicinal-solution storing section 20 is equal to or greater than the second water level L2. When determining in step S100 that the height of the liquid surface in the medicinal-solution storing section 20 has not reached the second water level L2 (NO in step S110), the endoscope reprocessor 1 shifts to step S190.

In step S190, the endoscope reprocessor 1 executes, via the output section 8, an output for requesting the user to supply the medicinal solution into the medicinal-solution storing section 20. After executing step S190, the endoscope reprocessor 1 returns to step S10. That is, the endoscope reprocessor 1 of the present embodiment does not start the reprocessing for the endoscope when the medicinal solution is not stored to the second water level L2 in the medicinal-solution storing section 20.

When determining in step S100 that the height of the liquid surface in the medicinal-solution storing section 20 is equal to or greater than the second water level L2 (YES in step S110), the endoscope reprocessor 1 shifts to step S120.

Instep S120, the endoscope reprocessor 1 executes concentration measurement of the medicinal solution with the concentration meter 80. Subsequently, in step S130, the endoscope reprocessor 1 determines whether a measurement value of the concentration of the medicinal solution is within a predetermined range. The predetermined range of the concentration is a range in which the medicinal solution exhibits medicinal effects necessary for executing the reprocessing.

In step S130, when determining that the measurement value of the concentration of the medicinal solution is within the predetermined range, the endoscope reprocessor 1 shifts to step S150 and executes the reprocessing for the endoscope. In the present embodiment, the reprocessing for the endoscope performed using the medicinal solution includes disinfecting treatment for introducing the medicinal solution into the treatment tank 2 and immersing the endoscope in the medicinal solution.

In the disinfecting treatment, after switching the switching valve 22 to the closed state, the endoscope reprocessor 1 operates the medicinal solution pump 27 and transfers the medicinal solution from the medicinal-solution storing section 20 to the treatment tank 2 in which the endoscope is disposed. After the medicinal solution is stored to a predetermined water level in the treatment tank 2, the endoscope reprocessor 1 stops the medicinal solution pump 27 and performs operation of the circulation pump 41 for a predetermined time period. After stopping the circulation pump 41, the endoscope reprocessor 1 switches the switching valve 22 to the state in which the discharge conduit 21 and the collection conduit 23 communicate and collects the medicinal solution in the treatment tank 2 into the medicinal-solution storing section 20. After an end of the reprocessing in step S150, the endoscope reprocessor 1 returns to step S10.

On the other hand, when determining in step S130 that the measurement value of the concentration of the medicinal solution is outside the predetermined range, the endoscope reprocessor 1 shifts to step S140. In step S140, the endoscope reprocessor 1 outputs, via the output section 8, a request for work for replacing the medicinal solution in the medicinal-solution storing section 20 with the unused medicinal solution. After executing step S140, the endoscope reprocessor 1 returns to step S10.

That is, the endoscope reprocessor 1 of the present embodiment does not start the reprocessing for the endoscope when the concentration of the medicinal solution stored in the medicinal-solution storing section 20 is outside the predetermined range.

On the other hand, when determining in step S40 that the instruction for executing the reprocessing is not inputted, the endoscope reprocessor 1 shifts to step S200.

In step S200, the endoscope reprocessor 1 checks, with the water level sensor 55, whether the height of the liquid surface in the medicinal-solution storing section 20 is equal to or greater than the third water level L3. When determining in step S200 that the height of the liquid surface in the medicinal-solution storing section 20 has not reached the third water level L3 (NO in step S210), the endoscope reprocessor 1 shifts to step S220 and executes a moisture retaining operation shown in the flowchart of Fig. 5.

A state in which the height of the liquid surface in the medicinal-solution storing section 20 has not reached the third water level L3 is a state in which the liquid in the medicinal-solution storing section 20 is discharged and the measurement surface 82 of the concentration meter 80 is exposed in the air. The moisture retaining operation in step S220 is explained below. After executing step S220, the endoscope reprocessor 1 returns to step S10.

When determining in step S200 that the height of the liquid surface in the medicinal-solution storing section 20 is equal to or greater than the third water level L3 (YES in step S210), the endoscope reprocessor 1 returns to step S10.

A power saving mode, to which the endoscope reprocessor 1 shifts according to execution of step S300, is explained.

As shown in Fig. 4, in the power saving mode, first, in step S310, the endoscope reprocessor 1 determines whether an instruction for power-on is inputted by the user. When determining in step S310 that the instruction for power-on is inputted, the endoscope reprocessor 1 shifts to step S350, starts the main routine shown in Fig. 3, and ends the power saving mode.

On the other hand, when determining in step S310 that the instruction for power-on is not inputted, the endoscope reprocessor 1 shifts to step S320.

In step S320, the endoscope reprocessor 1 checks, with the water level sensor 55, whether the height of the liquid surface in the medicinal-solution storing section 20 is equal to or greater than the third water level L3. When determining in step S320 that the height of the liquid surface in the medicinal-solution storing section 20 has not reached the third water level L3 (NO in step S330), the endoscope reprocessor 1 shifts to step S340 and executes the moisture retaining operation shown in the flowchart of Fig. 5. The moisture retaining operation in step S340 is explained below. After executing step S340, the endoscope reprocessor 1 returns to step S310.

The moisture retaining operation executed in step S220 and step S340 is explained.

As shown in Fig. 5, in the moisture retaining operation, first, in step S410, the endoscope reprocessor 1 recognizes an exposure time period T1 in which the measurement surface 82 of the concentration meter 80 is exposed in the air after coming into contact with the liquid.

The exposure time period T1 is a value of a shorter one of an elapsed time period from a point in time when the discharge process (step S80) for discharging the medicinal solution from the medicinal-solution storing section 20 with the liquid discharge section 28 is executed last and an elapsed time period from a point in time when liquid supply operation for supplying the liquid to the measurement surface 82 with the liquid supply section 60 is executed last. The exposure time period T1 is calculated from operation history information of the endoscope reprocessor 1 stored by the control section 5.

When determining that the exposure time period T1 recognized in step S410 is equal to or longer than a predetermined time period Tth (YES in step S420), the endoscope reprocessor 1 shifts to step S430 and drives the liquid supply section 60 to execute the liquid supply operation for supplying the liquid to the measurement surface 82. In the liquid supply operation, the endoscope reprocessor 1 continues operation of the discharge pump 64 for a predetermined time period and discharges the medicinal solution stored in the liquid holding section 62 from the nozzle 61 toward the measurement surface 82. According to execution of step S430, the measurement surface 82 of the permeation membrane 86 included in the concentration meter 80 is in contact with the medicinal solution for a predetermined time period. Therefore, the measurement surface 82 changes to the wet state. After executing step S430, the endoscope reprocessor 1 ends the moisture retaining operation.

On the other hand, when determining that the exposure time period T1 recognized in step S410 is shorter than the predetermined time period Tth (NO in step S420), the endoscope reprocessor 1 skips step S430 and ends the moisture retaining operation.

As explained above, the endoscope reprocessor 1 of the present embodiment includes the medicinal-solution storing section 20 that stores the medicinal solution, the concentration meter 80 in which the measurement surface 82 of the permeation membrane 86 is disposed at the predetermined first water level L1 in the medicinal-solution storing section 20, the liquid discharge section 28 that discharges the medicinal solution from the medicinal-solution storing section 20, the liquid supply section 60 that supplies the liquid to the permeation membrane 86 of the concentration meter 80, and the control section 5 connected to the liquid discharge section 28 and the liquid supply section 60, the control section 5 driving the liquid supply section to wet the permeation membrane 86 when the liquid surface of the medicinal solution in the medicinal-solution storing section 20 is lower than the first water level L1.

With the endoscope reprocessor 1 of the present embodiment, when the liquid surface of the medicinal solution in the medicinal-solution storing section 20 is lower than the first water level L1 and the measurement surface 82 of the concentration meter 80 is exposed in the air, since the endoscope reprocessor 1 drives the liquid supply section 60 to wet the measurement surface 82, the measurement surface 82 is kept in the wet state in which concentration measurement can be executed.

Therefore, even when a state in which the medicinal solution is absent in the medicinal-solution storing section 20 is kept for a relatively long period, for example, when the medicinal solution is discharged from the medicinal-solution storing section 20 on weekends and a new medicinal solution is supplied into the medicinal-solution storing section 20 on a weekday, the endoscope reprocessor 1 of the present embodiment can execute the concentration measurement of the medicinal solution by the concentration meter 80 without delay after preparing the medicinal solution and start the following reprocessing.

In the present embodiment, when the exposure time period T1 in which the measurement surface 82 of the concentration meter 80 is exposed in the air is equal to or longer than the predetermined time period Tth, the endoscope reprocessor 1 drives the liquid supply section 60 to wet the measurement surface 82. In other words, in the present embodiment, the control section 5 intermittently drives the liquid supply section 60 in a period in which the measurement surface 82 of the concentration meter 80 is exposed in the air. Therefore, it is possible to reduce a consumed amount of electric power necessary for driving the liquid supply section 60.

Note that the liquid supply section 60 of the present embodiment includes the nozzle 61 for directly blowing the liquid toward the measurement surface 82 of the concentration meter 80. However, the liquid supply section 60 may cause the liquid discharged from the nozzle 61 to flow along, for example, an outer surface of the housing 81 or an inner wall surface of the medicinal-solution storing section 20 and thereafter cause the liquid to reach the measurement surface 82.

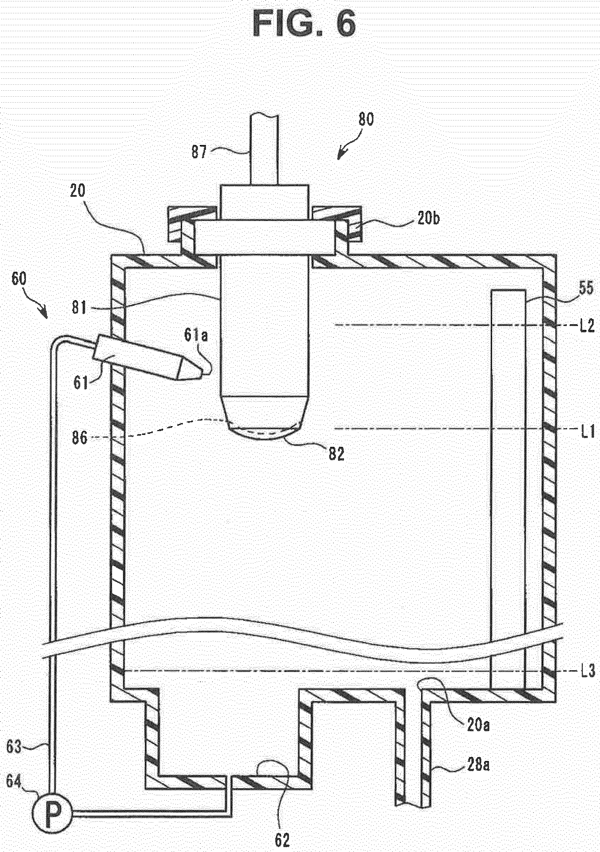

In Fig. 6, a modification of the liquid supply section 60 of the present embodiment is shown. The liquid supply section 60 of the modification includes the nozzle 61 in which the opening 61a is disposed above the measurement surface 82 of the permeation membrane 86 in a gravity direction. The opening 61 a of the nozzle 61 opens toward the outer surface of the housing 81 of the concentration meter 80.

In the modification, the liquid discharged from the opening 61 a of the nozzle 61 by driving of the liquid supply section 60 flows downward along the outer surface of the housing 81 and thereafter comes into contact with the measurement surface 82.

In the modification as well, when the measurement surface 82 of the concentration meter 80 is exposed in the air, by driving the liquid supply section 60 to wet the measurement surface 82, it is possible to keep the measurement surface 82 in the wet state in which concentration measurement can be executed.

Note that, as shown in the figure, in the modification, by forming the measurement surface 82 as a curved surface convex downward, for example, a substantially spherical surface, it is possible to cause the liquid supplied from the liquid supply section 60 to flow along the entire measurement surface 82. Note that the measurement surface 82 may be planar.

In the modification, by forming an outer surface of a lower end portion, where the measurement surface 82 is provided, of the housing 81 in a taper shape reduced in diameter toward the measurement surface, the liquid supplied from the liquid supply section 60 is allowed to easily flow from the outer surface of the housing 81 to the measurement surface 82. Note that the outer surface of the housing 81 does not have to be the taper shape.

### (Second Embodiment)

A second embodiment of the present invention is explained. In the following explanation, only differences from the first embodiment are explained. Components same as the components in the first embodiment are denoted by the same reference numerals and signs and explanation of the components is omitted as appropriate.

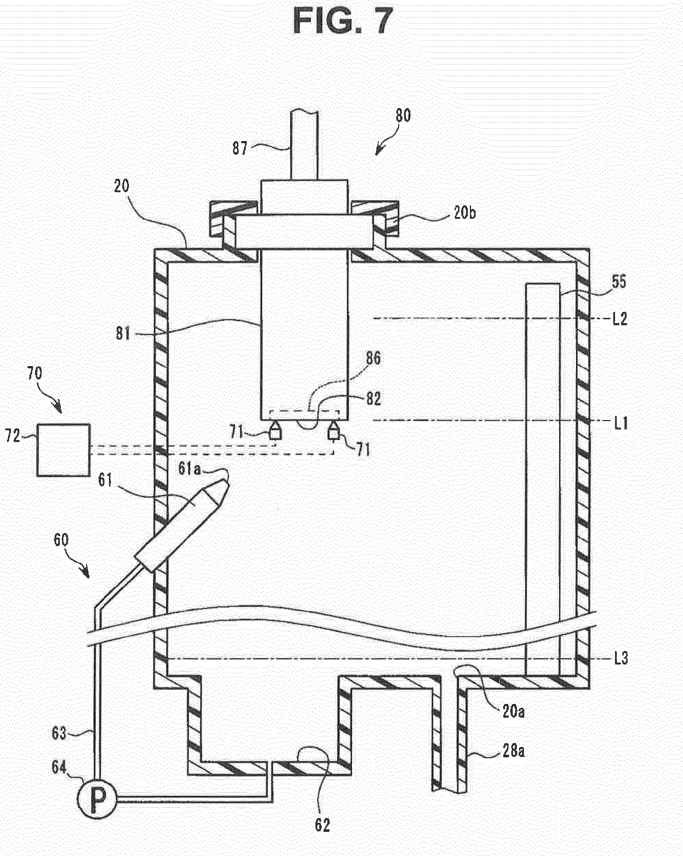

As shown in Fig. 7, the endoscope reprocessor 1 of the present embodiment includes a detecting section 70 that detects whether the measurement surface 82 of the permeation membrane 86 of the concentration meter 80 is in the wet state.

In the present embodiment, as an example, the detecting section 70 includes a pair of electrodes 71 and a measuring section 72 that measures an electric resistance value between the pair of electrodes 71. The pair of electrodes 71 is spaced apart by a predetermined distance and in contact with the measurement surface 82 of the permeation membrane 86. That is, the measuring section 72 measures an electric resistance value of the measurement surface 82 of the permeation membrane 86.

The measuring section 72 is electrically connected to the control section 5. Operation of the measuring section 72 is controlled by the control section 5. Information concerning the electric resistance value of the measurement surface 82 measured by the measuring section 72 is inputted to the control section 5.

The measurement of the electric resistance value of the measurement surface 82 by the measuring section 72 is performed when the measurement surface 82 is exposed in the air. That is, the measurement of the electric resistance value of the measurement surface 82 by the measuring section 72 is performed when the liquid surface in the medicinal-solution storing section 20 is lower than the first water level L1.

When the electric resistance value of the measurement surface 82 measured by the measuring section 72 is equal to or smaller than a predetermined value, the control section 5 determines that the measurement surface 82 is in the wet state. When the electric resistance value of the measurement surface 82 measured by the measuring section 72 exceeds the predetermined value, the control section 5 determines that the measurement surface 82 is in the dried state.

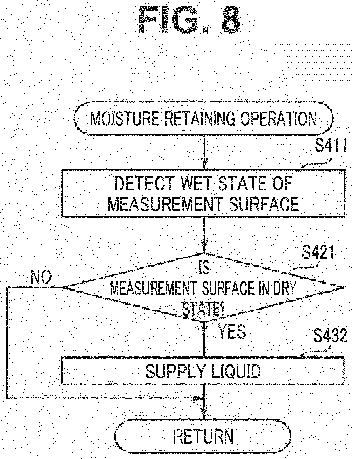

Fig. 8 is a flowchart of the moisture retaining operation of the endoscope reprocessor 1 of the present embodiment. In the moisture retaining operation of the present embodiment, first, in step 411, the endoscope reprocessor 1 drives the detecting section 70 and detects whether the measurement surface 82 of the permeation membrane 86 of the concentration meter 80 is in the wet state.

When determining in step S411 that the measurement surface 82 is in the dried state (YES in step S421), the endoscope reprocessor 1 shifts to step S431 and drives the liquid supply section 60 to execute the liquid supply operation for supplying the liquid to the measurement surface 82. In the liquid supply operation, the endoscope reprocessor 1 continues the operation of the discharge pump 64 for a predetermined time period and discharges the medicinal solution stored in the liquid holding section 62 from the nozzle 61 toward the measurement surface 82. According to execution of step S431, the measurement surface 82 of the permeation membrane 86 included in the concentration meter 80 is in contact with the medicinal solution for a predetermined time period. Therefore, the measurement surface 82 changes to the wet state. After executing step S431, the endoscope reprocessor 1 ends the moisture retaining operation.

On the other hand, when determining in step S411 that the measurement surface 82 is in the wet state (NO in step S421), the endoscope reprocessor 1 skips step S431 and ends the moisture retaining operation.

Other components and operations of the endoscope reprocessor 1 of the present embodiment are the same as the components and the operations in the first embodiment.

With the endoscope reprocessor 1 of the present embodiment, when the liquid surface of the medicinal solution in the medicinal-solution storing section 20 is lower than the first water level L1 and the measurement surface 82 of the concentration meter 80 is exposed in the air, since the endoscope reprocessor 1 drives the liquid supply section 60 to wet the measurement surface 82, the measurement surface 82 is kept in the wet state in which concentration measurement can be executed.

Therefore, as in the first embodiment, even when a state in which the medicinal solution is absent in the medicinal-solution storing section 20 is kept for a relatively long period, for example, when the medicinal solution is discharged from the medicinal-solution storing section 20 on weekends and a new medicinal solution is supplied into the medicinal-solution storing section 20 on a weekday, the endoscope reprocessor 1 of the present embodiment can execute the concentration measurement of the medicinal solution by the concentration meter 80 without delay after preparing the medicinal solution and start the following reprocessing.

In the present embodiment, the endoscope reprocessor 1 drives the liquid supply section 60 only when the measurement surface 82 is exposed in the air and the detecting section 70 detects that the measurement surface 82 is in the dried state. Therefore, it is possible to reduce a consumed amount of electric power necessary for driving the liquid supply section 60. In the present embodiment, it is possible to surely keep the measurement surface 82 in the wet state without being affected by changes in temperature, humidity, and the like of an ambient atmosphere in which the endoscope reprocessor 1 is placed.

Note that a configuration of the detecting section 70 only has to be capable of detecting whether the measurement surface 82 is in the wet state and is not limited to the present embodiment.

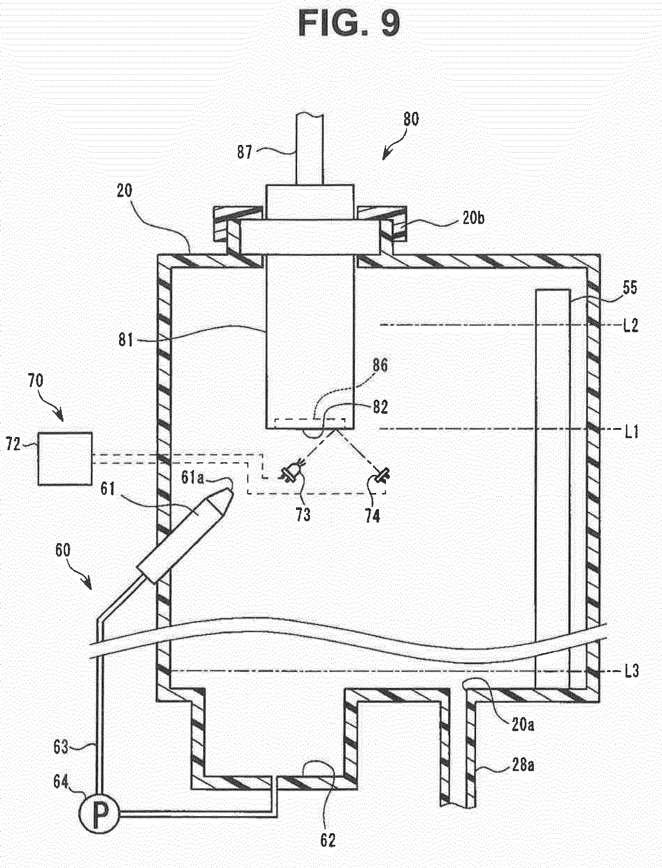

Fig. 9 is a diagram showing a first modification of the detecting section 70 of the present embodiment. The detecting section 70 of the first modification includes a light emitting section 73 such as an LED and a light receiving section 74 such as a photodiode. The light emitting section 73 and the light receiving section 74 are electrically connected to the measuring section 72.

The detecting section 70 of the first modification emits light from the light emitting section 73 toward the measurement surface 82 of the permeation membrane 86 and measures, in the light receiving section 74, intensity of the light reflected on the measurement surface 82. That is, the detecting section 70 measures reflectance of the light of the measurement surface 82. The measurement of the reflectance of the light of the measurement surface 82 by the detecting section 70 is performed when the measurement surface 82 is exposed in the air.

When the reflectance of the light of the measurement surface 82 measured by the detecting section 70 is equal to or larger than a predetermined value, the control section 5 of the first modification determines that the measurement surface 82 is in the wet state. When the reflectance of the light of the measurement surface 82 measured by the detecting section 70 is smaller than the predetermined value, the control section 5 determines that the measurement surface 82 is in the dried state.

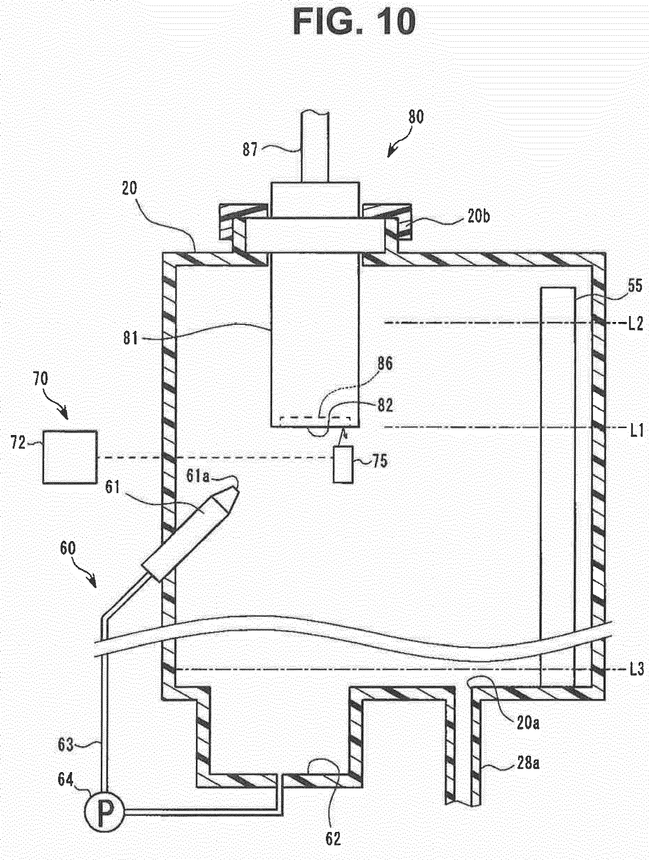

Fig. 10 is a diagram showing a second modification of the detecting section 70 of the present embodiment. The detecting section 70 of the second modification includes a temperature sensor 75 that measures temperature of the measurement surface 82 of the permeation membrane 86 such as an infrared thermometer.

The temperature sensor 75 is electrically connected to the measuring section 72. A measurement result of the temperature of the measurement surface 82 is inputted to the control section 5. The measurement of the temperature of the measurement surface 82 by the detecting section 70 is performed when the measurement surface 82 is exposed in the air.

When the temperature of the measurement surface 82 measured by the detecting section 70 is lower than ambient temperature, the control section 5 of the second modification determines that the measurement surface 82 is in the wet state. This is because, if the measurement surface 82 is in the wet state, since the liquid evaporates from the measurement surface 82, the temperature of the measurement surface 82 is lower than the ambient temperature. When the temperature of the measurement surface 82 measured by the detecting section 70 is equal to or higher than the ambient temperature, the control section 5 determines that the measurement surface 82 is in the dried state.

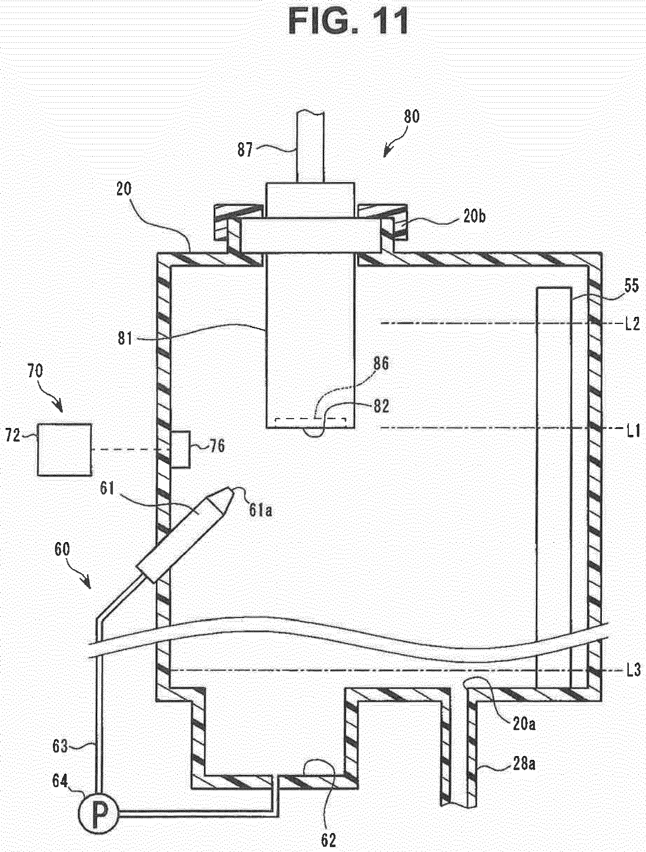

Fig. 11 is a diagram showing a third modification of the detecting section 70 of the present embodiment. The detecting section 70 of the third modification includes a humidity sensor 76 that measures humidity of the air in the medicinal-solution storing section 20.

The humidity sensor 76 is electrically connected to the measuring section 72. A measurement result of the humidity of the air in the medicinal-solution storing section 20 is inputted to the control section 5. The measurement of the humidity by the detecting section 70 is performed when the measurement surface 82 is exposed in the air.

When the humidity in the medicinal-solution storing section 20 measured by the detecting section 70 is equal to or larger than a predetermined value, the control section 5 of the third modification determines that the measurement surface 82 is in the wet state. When the humidity in the medicinal-solution storing section 20 measured by the detecting section 70 is smaller than the predetermined value, the control section 5 determines that the measurement surface 82 is in the dried state.

In the endoscope reprocessor 1 including the detecting section 70 of the first to third modifications, effects same as the effects of the present embodiment are obtained.

### (Third Embodiment)

A third embodiment of the present invention is explained. In the following explanation, only differences from the first and second embodiments are explained. Components same as the components in the first and second embodiments are denoted by the same reference numerals and signs and explanation of the components is omitted as appropriate.

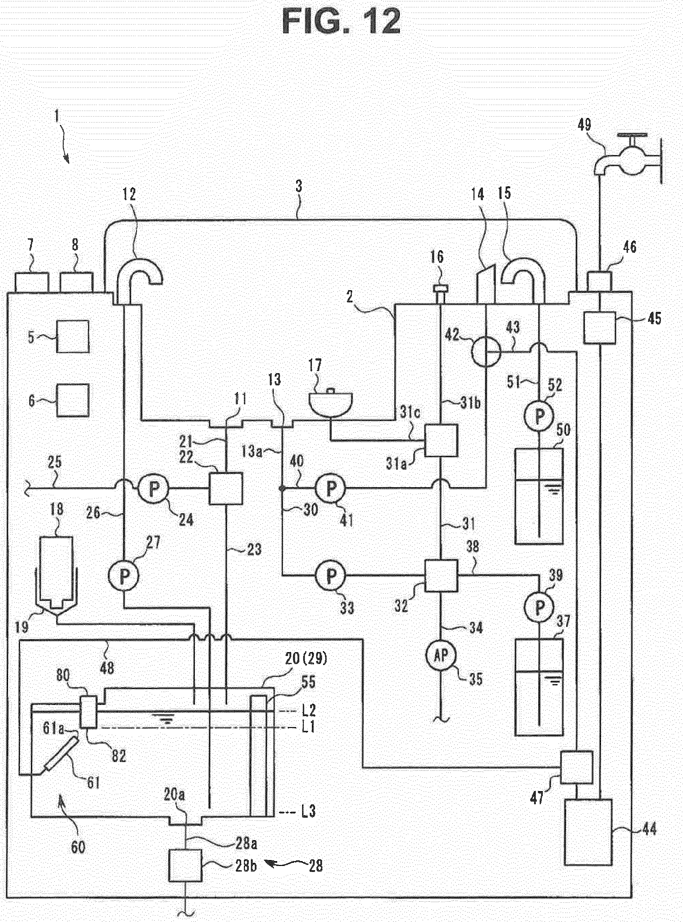

The present embodiment is different from the first and second embodiments in a configuration of the liquid supply section 60. As shown in Fig. 12, the liquid supply section 60 of the present embodiment communicates with the dilution conduit 48 for introducing water into the medicinal-solution storing section 20.

The liquid supply section 60 of the present embodiment supplies water supplied from the water supply source 49 to the measurement surface 82 of the concentration meter 80. The water supply source 49 is water equipment that supplies water at predetermined pressure. Therefore, the liquid supply section 60 of the present embodiment can discharge the water, which is liquid, from the opening 61a of the nozzle 61 without using a pump. That is, since driving of the liquid supply section 60 of the present embodiment is only switching operation of the water introducing valve 45 and the dilution valve 47, it is possible to execute the driving with a small power consumption amount.

Other components and operations of the endoscope reprocessor 1 of the present embodiment are the same as the components and the operations in the first and second embodiments.

With the endoscope reprocessor 1 of the present embodiment, when the liquid surface of the medicinal solution in the medicinal-solution storing section 20 is lower than the first water level L1 and the measurement surface 82 of the concentration meter 80 is exposed in the air, since the endoscope reprocessor 1 drives the liquid supply section 60 to wet the measurement surface 82, the measurement surface 82 is kept in the wet state in which concentration measurement can be executed.

Therefore, as in the first and second embodiments, even when a state in which the medicinal solution is absent in the medicinal-solution storing section 20 is kept for a relatively long period, for example, when the medicinal solution is discharged from the medicinal-solution storing section 20 on weekends and a new medicinal solution is supplied into the medicinal-solution storing section 20 on a weekday, the endoscope reprocessor 1 of the present embodiment can execute the concentration measurement of the medicinal solution by the concentration meter 80 without delay after preparing the medicinal solution and start the following reprocessing.

As in the second embodiment, the endoscope reprocessor 1 of the present embodiment may include the detecting section 70 that detects whether the measurement surface 82 is in the wet state. When the endoscope reprocessor 1 of the present embodiment includes the detecting section 70, the endoscope reprocessor 1 has effects same as the effects of the second embodiment.

### (Fourth Embodiment)

A fourth embodiment of the present invention is explained. In the following explanation, only differences from the first embodiment are explained. Components same as the components in the first embodiment are denoted by the same reference numerals and signs and explanation of the components is omitted as appropriate.

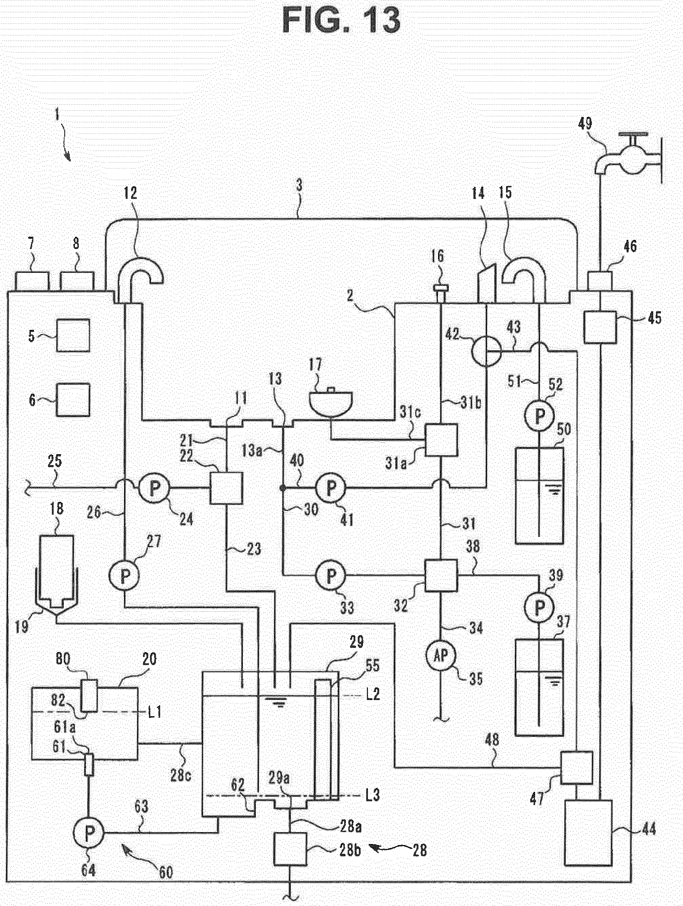

In the endoscope reprocessor 1 of the present embodiment shown in Fig. 13, the medicinal-solution storing section 20 is provided as a member different from the medicinal-solution collecting section 29.

The medicinal-solution collecting section 29 stores a medicinal solution more than an amount at least necessary for execution of reprocessing by the endoscope reprocessor 1. The medicinal-solution collecting section 29 communicates with the bottle connecting section 19, the collection conduit 23, the medicinal solution conduit 26, the dilution conduit 48, and the drain conduit 28a. The drain conduit 28a communicates with the liquid discharge port 20a provided on the bottom surface of the medicinal-solution collecting section 29 or near the bottom surface. These conduits are components same as the conduits in the first embodiment.

In the medicinal-solution collecting section 29, the water level sensor 55 and the liquid holding section 62 are provided. The water level sensor 55 at least detects whether a liquid surface in the medicinal-solution collecting section 29 has reached the second water level L2 explained above and whether a liquid surface of the medicinal solution has reached the third water level L3 explained below. The second water level L2 is height of the liquid surface of the medicinal solution in the case in which a volume of the medicinal solution at least necessary for the execution of the reprocessing is stored in the medicinal-solution collecting section 29. The third water level L3 is height near the bottom surface of the medicinal-solution collecting section 29.

The liquid holding section 62 is a concave section provided on the bottom surface of the medicinal-solution collecting section 29 and opening upward. The liquid holding section 62 opens in a position different from a liquid discharge port 29a. Therefore, even after the liquid in the medicinal-solution collecting section 29 is discharged from the liquid discharge port 29a, the liquid is stored in the liquid holding section 62. Note that, although not shown in the figure, the endoscope reprocessor 1 includes a component such as a cock for discharging the liquid stored in the liquid holding section 62 to the outside of the apparatus.

The medicinal-solution storing section 20 of the present embodiment communicates with the medicinal-solution collecting section 29 via the supply conduit 63 and a return conduit 28c. The supply conduit 63 causes the liquid holding section 62 of the medicinal-solution collecting section 29 and the nozzle 61 including the opening 61a disposed in the medicinal-solution storing section 20 to communicate. The discharge pump 64 is provided in the supply conduit 63.

The discharge pump 64 of the discharge pump 64 is operated, whereby the liquid stored in the liquid holding section 62 is discharged from the opening 61a of the nozzle 61 and introduced into the medicinal-solution storing section 20. The liquid introduced into the medicinal-solution storing section 20 returns to the medicinal-solution collecting section 29 through the return conduit 28c.

The measurement surface 82 of the concentration meter 80 is disposed at the predetermined first water level L1 in the medicinal-solution storing section 20. The medicinal-solution storing section 20 can store the medicinal solution to above the first water level L1.

The liquid discharge section 28 of the present embodiment discharges the liquid such as the medicinal solution or the water from the medicinal-solution storing section 20 and the medicinal-solution collecting section 29. A configuration for discharging the liquid from the medicinal-solution collecting section 29 is the same as the configuration in the first to third embodiments. That is, the liquid discharge section 28 includes the drain conduit 28a, the drain valve 28b, the medicinal solution conduit 26, and the medicinal solution pump 27. When an instruction for discharging the liquid from the medicinal-solution collecting section 29 is inputted via the operation section 7, first, the endoscope reprocessor 1 of the present embodiment starts operation of the medicinal solution pump 27 and stops the medicinal solution pump 27 at a point in time when it is detected by the water level sensor 55 that the height of the liquid surface in the medicinal-solution collecting section 29 is less than the third water level L3. In the operation, the liquid is discharged from the medicinal solution nozzle 12. The user can discharge the liquid remaining in the medicinal-solution collecting section 29 through the drain conduit 28a at a point in time of the stop of the medicinal solution pump 27 by switching the drain valve 28b to the open state.

The medicinal-solution storing section 20 communicates with the medicinal-solution collecting section 29 via the return conduit 28c. Therefore, if the liquid in the medicinal-solution collecting section 29 is discharged by the liquid discharge section 28, the liquid in the medicinal-solution storing section 20 is also discharged through the return conduit 28c and the medicinal-solution collecting section 29. That is, the liquid discharge section 28 of the present embodiment includes the return conduit 28c.

The liquid supply section 60 of the present embodiment is a component same as the liquid supply section 60 of the first embodiment. That is, after the liquid is discharged from the medicinal-solution storing section 20 and the medicinal-solution collecting section 29 by the liquid discharge section 28, the liquid supply section 60 supplies the liquid stored in the liquid holding section 62 to the measurement surface 82 of the permeation membrane 86 of the concentration meter 80.

In the embodiment shown in the figure, as an example, the opening 61a of the nozzle 61 of the liquid supply section 60 opens toward the measurement surface 82. At least a part of the liquid discharged from the opening 61 a of the nozzle 61 is blown against the measurement surface 82 and wets the measurement surface 82. Note that, as explained in the modification of the first embodiment, the nozzle 61 may be disposed at a position for causing the discharged liquid to flow along, for example, the outer surface of the housing 81 or the inner wall surface of the medicinal-solution storing section 20 and thereafter reach the measurement surface 82.

In the endoscope reprocessor 1 of the present embodiment, when concentration measurement of the medicinal solution by the concentration meter 80 is performed, prior to execution of the concentration measurement, the discharge pump 64 is operated to transfer the medicinal solution in the medicinal-solution collecting section 29 into the medicinal-solution storing section 20.

Operation of the endoscope reprocessor 1 of the present embodiment is the same as the operation explained with reference to Fig. 3 to Fig. 5 in the first embodiment. That is, the operation of the endoscope reprocessor 1 of the present embodiment is the same as the operation in the first embodiment.

Therefore, with the endoscope reprocessor 1 of the present embodiment, when the liquid is discharged from the medicinal-solution collecting section 29 and the medicinal-solution storing section 20 and the measurement surface 82 of the concentration meter 80 is exposed in the air, since the endoscope reprocessor 1 drives the liquid supply section 60 to wet the measurement surface 82, the measurement surface 82 is kept in the wet state in which the concentration measurement can be executed.

Therefore, as in the first embodiment, even when a state in which the medicinal solution is absent in the medicinal-solution storing section 20 is kept for a relatively long period, for example, when the medicinal solution is discharged from the medicinal-solution storing section 20 on weekends and a new medicinal solution is supplied into the medicinal-solution storing section 20 on a weekday, the endoscope reprocessor 1 of the present embodiment can execute the concentration measurement of the medicinal solution by the concentration meter 80 without delay after preparing the medicinal solution and start the following reprocessing.

In the present embodiment, when the exposure time period T1 in which the measurement surface 82 of the concentration meter 80 is exposed in the air is equal to or longer than the predetermined time period Tth, the endoscope reprocessor 1 drives the liquid supply section 60 to wet the measurement surface 82. In other words, in the present embodiment, the control section 5 intermittently drives the liquid supply section 60 in a period in which the measurement surface 82 of the concentration meter 80 is exposed in the air. Therefore, it is possible to reduce a consumed amount of electric power necessary for driving the liquid supply section 60.

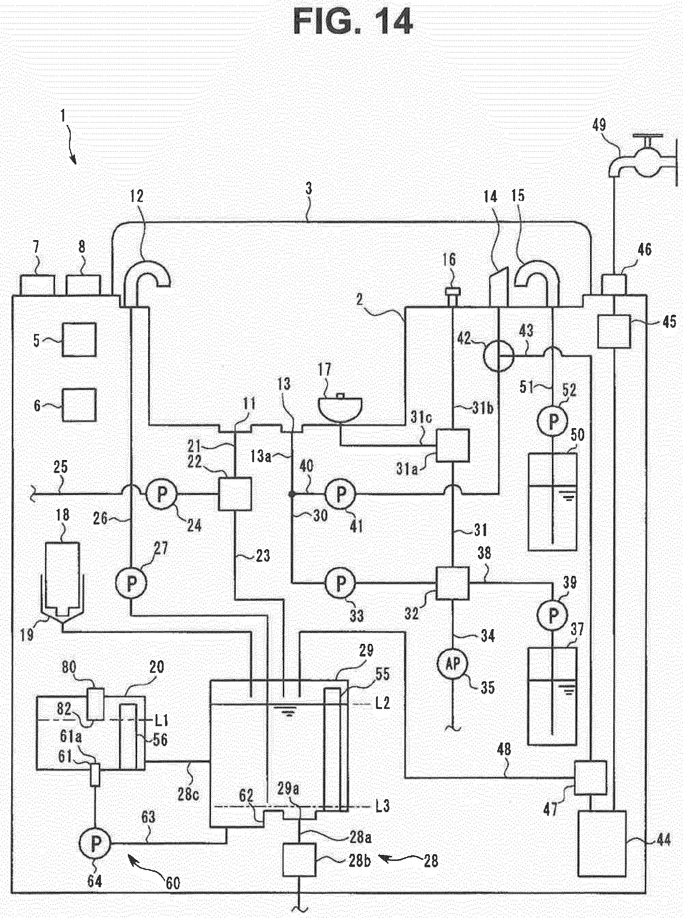

A modification of the endoscope reprocessor 1 of the present embodiment is shown in Fig. 14. The endoscope reprocessor 1 of the modification includes a water level sensor 56 in the medicinal-solution storing section 20. The water level sensor 56 detects whether the height of the liquid surface of the medicinal-solution storing section 20 has reached the first water level L1. The water level sensor 56 is electrically connected to the control section 5. A detection result by the water level sensor 56 is inputted to the control section 5.

A configuration of the water level sensor 56 is not particularly limited. The water level sensor 56 may be a so-called electrode-type water level sensor including, for example, a plurality of electrodes disposed spaced apart from one another, the electrode-type water level sensor detecting, based on presence or absence of electric conduction among the plurality of electrodes that changes according to whether the plurality of electrodes are sunk in liquid, whether the liquid surface has reached a predetermined water level. For example, the water level sensor 56 may be a so-called float-type water level sensor that detects, based on an operation state of a switch that opens and closes according to up and down movements of a float floating on the medicinal solution, whether the liquid surface has reached the predetermined water level.

In the endoscope reprocessor 1 of the modification, when performing the concentration measurement of the medicinal solution by the concentration meter 80, prior to execution of the concentration measurement, the endoscope reprocessor 1 operates the discharge pump 64 and transfers the medicinal solution in the medicinal-solution collecting section 29 into the medicinal-solution storing section 20. When it is detected by the water level sensor 56 that the liquid surface in the medicinal-solution storing section 20 is located above the first water level L1, the endoscope reprocessor 1 executes the concentration measurement of the medicinal solution by the concentration meter 80.

The modification is different from the embodiments described above in the liquid supply operation in step S430. In the liquid supply operation of the modification, the endoscope reprocessor 1 continues the operation of the discharge pump 64 until it is detected that the height of the liquid surface in the medicinal-solution storing section 20 is equal to or greater than the first water level L1. That is, in the liquid supply operation of the modification, the endoscope reprocessor 1 immerses the measurement surface 82 of the permeation membrane 86 of the concentration meter 80 in the liquid.

Other components and other operations of the endoscope reprocessor 1 of the modification are the same as the components and the operations in the embodiments described above.

Therefore, in the modification as well, when the measurement surface 82 of the concentration meter 80 is exposed in the air, by driving the liquid supply section 60 to wet the measurement surface 82, it is possible to keep the measurement surface 82 in the wet state in which concentration measurement can be executed.

### (Fifth Embodiment)

A fifth embodiment of the present invention is explained. In the following explanation, only differences from the fourth embodiment are explained. Components same as the components in the fourth embodiment are denoted by the same reference numerals and signs and explanation of the components is omitted as appropriate.

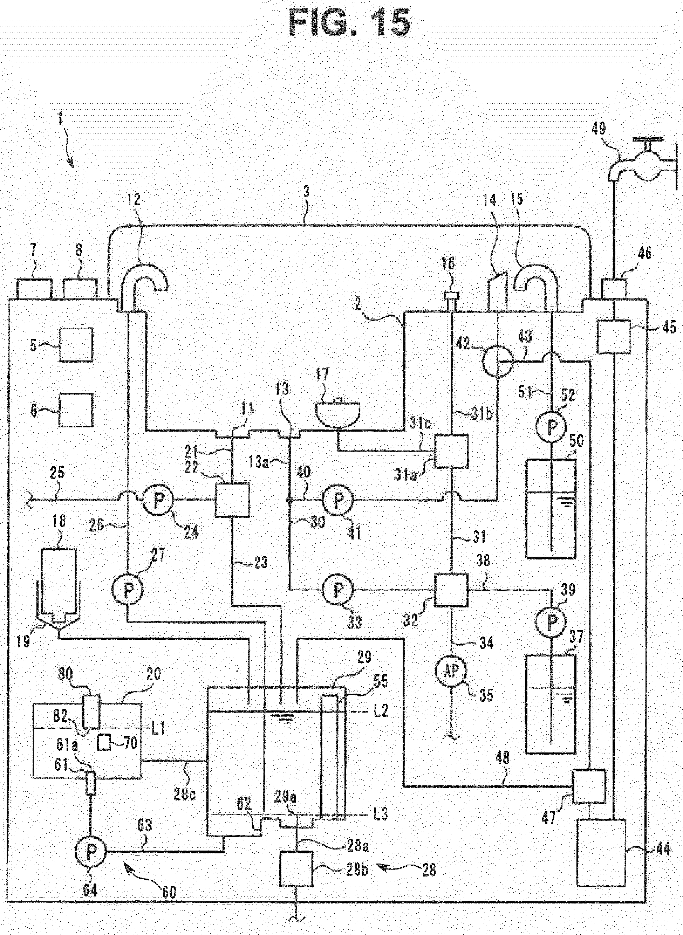

The endoscope reprocessor 1 of the present embodiment shown in Fig. 15 includes the detecting section 70 that detects whether the measurement surface 82 of the permeation membrane 86 of the concentration meter 80 is in the wet state. A configuration of the detecting section 70 is not particularly limited as explained in the second embodiment.

For example, the detecting section 70 may be configured to measure an electric resistance value of the measurement surface 82, may be configured to measure reflectance of light of the measurement surface 82, may be configured to measure a temperature difference between the measurement surface 82 and ambient temperature, or may be configured to measure humidity in the medicinal-solution storing section 20.

The endoscope reprocessor 1 of the present embodiment is different from the fourth embodiment in a moisture retaining operation. The moisture retaining operation of the present embodiment is the same as the moisture retaining operation of the second embodiment explained with reference to Fig. 8.

That is, in the moisture retaining operation of the present embodiment, when the measurement surface 82 of the concentration meter 80 is exposed in the air and it is detected by the detecting section 70 that the measurement surface 82 is in the dried state, the endoscope reprocessor 1 drives the liquid supply section 60 to wet the measurement surface 82.

According to the present embodiment, as in the second embodiment, it is possible to keep the measurement surface 82 of the concentration meter 80 in the wet state in which concentration measurement can be executed.

Therefore, as in the second embodiment, even when a state in which the medicinal solution is absent in the medicinal-solution storing section 20 is kept for a relatively long period, for example, when the medicinal solution is discharged from the medicinal-solution storing section 20 on weekends and a new medicinal solution is supplied into the medicinal-solution storing section 20 on a weekday, the endoscope reprocessor 1 of the present embodiment can execute the concentration measurement of the medicinal solution by the concentration meter 80 without delay after preparing the medicinal solution and start the following reprocessing.

In the present embodiment, the endoscope reprocessor 1 drives the liquid supply section 60 only when the measurement surface 82 is exposed in the air and the detecting section 70 detects that the measurement surface 82 is in the dried state. Therefore, it is possible to reduce a consumed amount of electric power necessary for driving the liquid supply section 60. In the present embodiment, it is possible to surely keep the measurement surface 82 in the wet state without being affected by changes in temperature, humidity, and the like of an ambient atmosphere in which the endoscope reprocessor 1 is placed.

### (Sixth Embodiment)

A sixth embodiment of the present invention is explained. In the following explanation, only differences from the fourth and fifth embodiments are explained. Components same as the components in the fourth and fifth embodiments are denoted by the same reference numerals and signs and explanation of the components is omitted as appropriate.

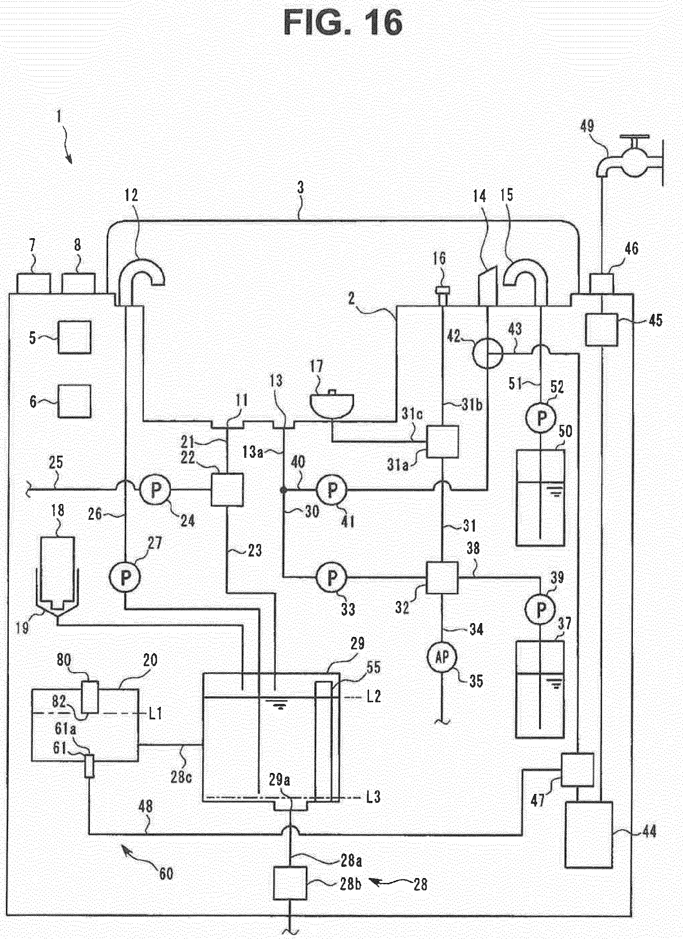

The endoscope reprocessor 1 of the present embodiment shown in Fig. 16 is different from the fourth and fifth embodiments in a configuration of the liquid supply section 60. The nozzle 61 of the liquid supply section 60 of the present embodiment communicates with the dilution conduit 48 for introducing water into the medicinal-solution collecting section 29.

The water supplied from the dilution conduit 48 is introduced into the medicinal-solution collecting section 29 through the nozzle 61, the medicinal-solution storing section 20, and the return conduit 28c. That is, the medicinal-solution storing section 20 of the present embodiment is provided on a route for introducing the water supplied from the water supply source 49 into the medicinal-solution collecting section 29.

The liquid supply section 60 of the present embodiment supplies the water supplied from the water supply source 49 to the measurement surface 82 of the concentration meter 80. The water supply source 49 is waterworks that supplies water at predetermined pressure. Therefore, the liquid supply section 60 of the present embodiment can discharge the water, which is liquid, from the opening 61a of the nozzle 61 without using a pump. That is, since driving of the liquid supply section 60 of the present embodiment is only switching operation of the water introducing valve 45 and the dilution valve 47, it is possible to execute the driving with a small power consumption amount.

Other components and operations of the endoscope reprocessor 1 of the present embodiment are the same as the components and the operations in the fourth and fifth embodiments.

With the endoscope reprocessor 1 of the present embodiment, when the measurement surface 82 of the concentration meter 80 is exposed in the air, since the endoscope reprocessor 1 drives the liquid supply section 60 to wet the measurement surface 82, the measurement surface 82 is kept in the wet state in which concentration measurement can be executed.

Therefore, as in the fourth and fifth embodiments, even when a state in which the medicinal solution is absent in the medicinal-solution storing section 20 is kept for a relatively long period, for example, when the medicinal solution is discharged from the medicinal-solution storing section 20 on weekends and a new medicinal solution is supplied into the medicinal-solution storing section 20 on a weekday, the endoscope reprocessor 1 of the present embodiment can execute the concentration measurement of the medicinal solution by the concentration meter 80 without delay after preparing the medicinal solution and start the following reprocessing.

As in the fifth embodiment, the endoscope reprocessor 1 of the present embodiment may include the detecting section 70 that detects whether the measurement surface 82 is in the wet state. When the endoscope reprocessor 1 of the present embodiment includes the detecting section 70, the endoscope reprocessor 1 has effects same as the effects of the fifth embodiment.

### (Seventh Embodiment)

A seventh embodiment of the present invention is explained. In the following explanation, only differences from the fourth embodiment are explained. Components same as the components in the fourth embodiment are denoted by the same reference numerals and signs and explanation of the components is omitted as appropriate.

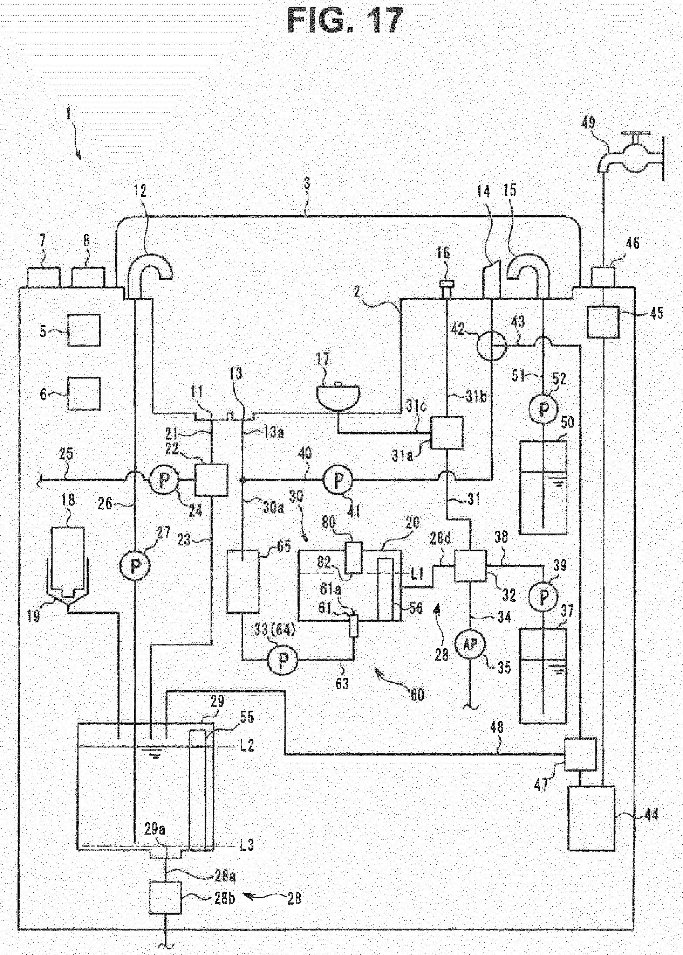

In the endoscope reprocessor 1 of the present embodiment shown in Fig. 17, the medicinal-solution storing section 20 is provided halfway in the endoscope circulation conduit 30.

As explained above, the endoscope circulation conduit 30 is a conduit branching from the circulation conduit 13a and connected to the channel valve 32. The endoscope circulation conduit 30 of the present embodiment is configured by concatenating a first conduit 30a, a liquid holding section 65, the supply conduit 63, the nozzle 61, the medicinal-solution storing section 20, and a second conduit 28d in order from a branching section of the circulation conduit 13a.

The circulation pump 33 is provided in the supply conduit 63. If the circulation pump 33 operates in a state in which liquid is stored in the treatment tank 2, the liquid in the treatment tank 2 is introduced into the channel valve 32 through the circulation conduit 13a, the first conduit 30a, the liquid holding section 65, the supply conduit 63, the nozzle 61, the medicinal-solution storing section 20, and the second conduit 28d.

The liquid holding section 65 stores a volume of liquid with which moisture retaining treatment can be continued for a predetermined time period. The predetermined time period in which the moisture retaining treatment should be continued is, for example, 72 hours longer than a time period from a Friday evening to a Monday morning. Note that the liquid holding section 65 may function as the first conduit 30a as well.

The measurement surface 82 of the concentration meter 80 is disposed at the predetermined first water level L1 in the medicinal-solution storing section 20. The medicinal-solution storing section 20 can store the medicinal solution to above the first water level L1. The liquid discharge section 28 of the present embodiment discharges the liquid such as the medicinal solution or the water from the medicinal-solution storing section 20. The liquid in the medicinal-solution storing section 20 is discharged from the second conduit 28d.

The water level sensor 56 is provided in the medicinal-solution storing section 20. The water level sensor 56 detects whether the height of the liquid surface in the medicinal-solution storing section 20 has reached the first water level L1. The water level sensor 56 is electrically connected to the control section 5. A detection result by the water level sensor 56 is inputted to the control section 5.

A configuration of the water level sensor 56 is not particularly limited. The water level sensor 56 may be a so-called electrode-type water level sensor including, for example, a plurality of electrodes disposed spaced apart from one another, the electrode-type water level sensor detecting, based on presence or absence of electric conduction among the plurality of electrodes that changes according to whether the plurality of electrodes are sunk in liquid, whether the liquid surface has reached a predetermined water level. For example, the water level sensor 56 may be a so-called float-type water level sensor that detects, based on an operation state of a switch that opens and closes according to up and down movements of a float floating on the medicinal solution, whether the liquid surface has reached the predetermined water level.

The liquid supply section 60 of the present embodiment supplies the liquid stored in the liquid holding section 65 to the measurement surface 82 of the permeation membrane 86 of the concentration meter 80. When reprocessing for the endoscope is executed, the liquid such as the water or the medicinal solution flows in the circulation conduit 30. Therefore, after the execution of the reprocessing, the liquid is stored in the liquid holding section 65.

In the embodiment shown in the figure, as an example, the opening 61a of the nozzle 61 of the liquid supply section 60 opens toward the measurement surface 82. At least a part of the liquid discharged from the opening 61a of the nozzle 61 is blown against the measurement surface 82 and wets the measurement surface 82. Note that, as explained in the modification of the first embodiment, the nozzle 61 may be disposed at a position for causing the discharged liquid to flow along, for example, the outer surface of the housing 81 or the inner wall surface of the medicinal-solution storing section 20 and thereafter reach the measurement surface 82.

In the endoscope reprocessor 1 of the present embodiment, when concentration measurement of the medicinal solution by the concentration meter 80 is performed, prior to execution of the concentration measurement, the medicinal solution pump 27 is operated to introduce the medicinal solution in the medicinal-solution collecting section 29 into the treatment tank 2. Thereafter, the circulation pump 33 is operated to introduce the medicinal solution in the treatment tank 2 into the medicinal-solution storing section 20 through the circulation port 13, the circulation conduit 13a, the first conduit 30a, the supply conduit 63, and the nozzle 61 and executes the concentration measurement.

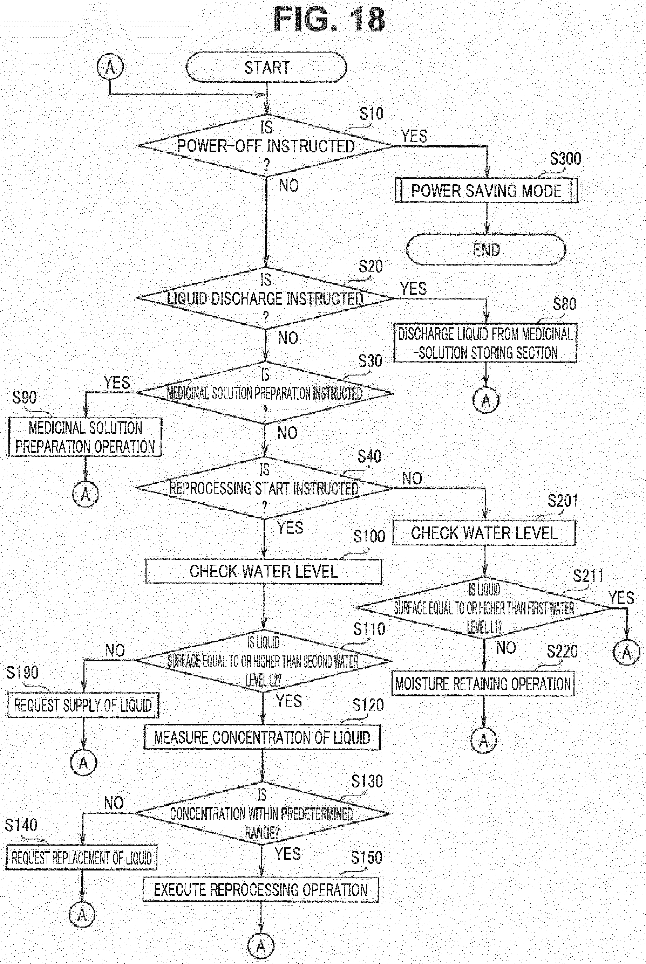

Operation of the endoscope reprocessor 1 is explained with reference to flowcharts shown in Fig. 18, Fig. 19, and Fig. 20. A flow of a main routine shown in Fig. 18 is started, for example, when the power supply of the endoscope reprocessor 1 is brought into an ON state. Note that an input of an operation instruction from the user to the endoscope reprocessor 1 is performed via the operation section 7.

In the main routine of the endoscope reprocessor 1 of the present embodiment, operation performed after it is determined in step S40 that the instruction for executing the reprocessing is not inputted (NO in step S40) is different from the operation explained in the first embodiment.

When determining in step S40 that the instruction for executing the reprocessing is not inputted, the endoscope reprocessor 1 shifts to step S201.

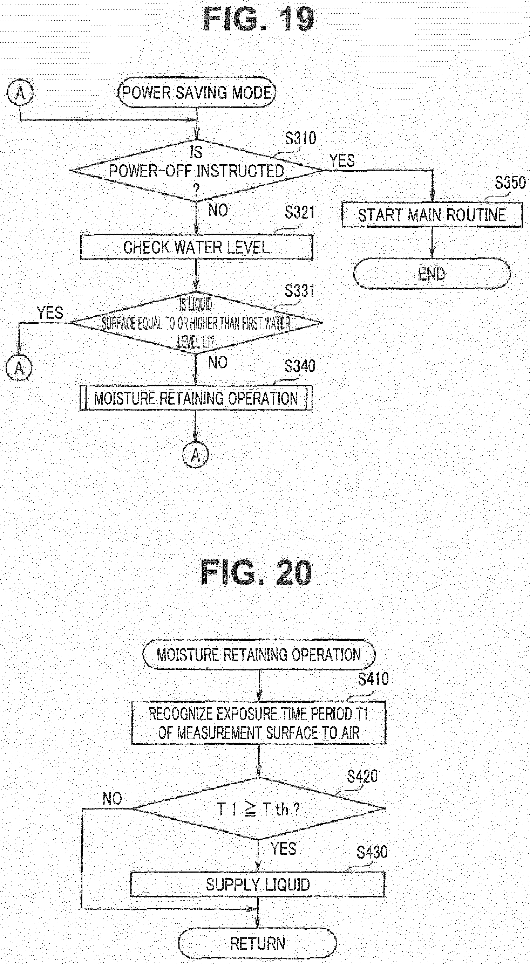

In step S201, the endoscope reprocessor 1 checks, with the water level sensor 56, whether the height of the liquid surface in the medicinal-solution storing section 20 is equal to or greater than the first water level L1. When determining in step S201 that the height of the liquid surface in the medicinal-solution storing section 20 has not reached the first water level L1 (NO in step S211), the endoscope reprocessor 1 shifts to step S220 and executes moisture retaining operation shown in the flowchart of Fig. 19.

A state in which the height of the liquid surface in the medicinal-solution storing section 20 has not reached the first water level L1 is a state in which the liquid in the medicinal-solution storing section 20 is discharged and the measurement surface 82 of the concentration meter 80 is exposed in the air. The moisture retaining operation in step S220 is explained below. After executing step S220, the endoscope reprocessor 1 returns to step S10.

When determining in step S201 that the height of the liquid surface in the medicinal-solution storing section 20 is equal to or greater than the first water level L1 (YES in step S211), the endoscope reprocessor 1 returns to step S10.

A power saving mode, to which the endoscope reprocessor 1 shifts according to execution of step S300 of the main routine, is explained. As shown in Fig. 19, in the power saving mode, first, in step S310, the endoscope reprocessor 1 determines whether an instruction for power-on is inputted by the user. When determining in step S310 that the instruction for power-on is inputted, the endoscope reprocessor 1 shifts to step S350, starts the main routine shown in Fig. 18, and ends the power saving mode.

On the other hand, when determining in step S310 that the instruction for power-on is not inputted, the endoscope reprocessor 1 shifts to step S321.

In step S321, the endoscope reprocessor 1 checks, with the water level sensor 56, whether the height of the liquid surface in the medicinal-solution storing section 20 is equal to or greater than the first water level L1. When determining in step S321 that the height of the liquid surface in the medicinal-solution storing section 20 has not reached the first water level L1 (NO in step S331), the endoscope reprocessor 1 shifts to step S340 and executes moisture retaining operation shown in the flowchart of Fig. 20. The moisture retaining operation in step S340 is explained below. After executing step S340, the endoscope reprocessor 1 returns to step S310.

The moisture retaining operation executed in step S220 and step S340 is explained. The moisture retaining operation is the same as the moisture retaining operation in the first embodiment.

As shown in Fig. 20, in the moisture retaining operation, first, in step S410, the endoscope reprocessor 1 recognizes the exposure time period T1 in which the measurement surface 82 of the concentration meter 80 is exposed in the air after coming into contact with the liquid.

The exposure time period T1 is a value of a shorter one of an elapsed time period from a point in time when the discharge process (step S80) for discharging the medicinal solution from the medicinal-solution storing section 20 with the liquid discharge section 28 is executed last and an elapsed time period from a point in time when a liquid supply operation for supplying the liquid to the measurement surface 82 with the liquid supply section 60 is executed last. The exposure time period T1 is calculated from operation history information of the endoscope reprocessor 1 stored by the control section 5.

When determining that the exposure time period T1 recognized in step S410 is equal to or longer than the predetermined time period Tth (YES in step S420), the endoscope reprocessor 1 shifts to step S430 and drives the liquid supply section 60 to execute the liquid supply operation for supplying the liquid to the measurement surface 82. In the liquid supply operation, the endoscope reprocessor 1 continues operation of the discharge pump 64 for a predetermined time period and discharges the medicinal solution stored in the liquid holding section 62 from the nozzle 61 toward the measurement surface 82. According to execution of step S430, the measurement surface 82 of the permeation membrane 86 included in the concentration meter 80 is in contact with the medicinal solution for a predetermined time period. Therefore, the measurement surface 82 changes to the wet state. After executing step S430, the endoscope reprocessor 1 ends the moisture retaining operation.

On the other hand, when determining that the exposure time period T1 recognized in step S410 is shorter than the predetermined time period Tth (NO in step S420), the endoscope reprocessor 1 skips step S430 and ends the moisture retaining operation.

As explained above, with the endoscope reprocessor 1 of the present embodiment, when the liquid surface of the medicinal solution in the medicinal-solution storing section 20 is lower than the first water level L1 and the measurement surface 82 of the concentration meter 80 is exposed in the air, since the endoscope reprocessor 1 drives the liquid supply section 60 to wet the measurement surface 82, the measurement surface 82 is kept in the wet state in which concentration measurement can be executed.

Therefore, even when a state in which the medicinal solution is absent in the medicinal-solution storing section 20 is kept for a relatively long period, for example, when the medicinal solution is discharged from the medicinal-solution storing section 20 on weekends and a new medicinal solution is supplied into the medicinal-solution storing section 20 on a weekday, the endoscope reprocessor 1 of the present embodiment can execute the concentration measurement of the medicinal solution by the concentration meter 80 without delay after preparing the medicinal solution and start the following reprocessing.

In the present embodiment, when the exposure time period T1 in which the measurement surface 82 of the concentration meter 80 is exposed in the air is equal to or longer than the predetermined time period Tth, the endoscope reprocessor 1 drives the liquid supply section 60 to wet the measurement surface 82. In other words, in the present embodiment, the control section 5 intermittently drives the liquid supply section 60 in a period in which the measurement surface 82 of the concentration meter 80 is exposed in the air. Therefore, it is possible to reduce a consumed amount of electric power necessary for driving the liquid supply section 60.

Note that the present invention is not limited to the embodiments explained above and can be changed as appropriate in a range not contrary to the gist or the idea of the invention read from claims and the entire specification. An endoscope reprocessor involving such a change is also included in the technical scope of the present invention.

The application is based upon and claims priority from Japanese Patent Application No. 2015-114993 filed in Japan on June 5, 2015, disclosed contents of which are incorporated in the specification, the claims, and the drawings of the application.

## Claims

1. An endoscope reprocessor comprising:
a concentration meter including a housing, an electrode housed in the housing, a permeation membrane held by the housing, and internal liquid encapsulated in the housing, the concentration meter measuring concentration of a medicinal solution;
a medicinal-solution storing section including a holding section configured to hold the concentration meter such that the permeation membrane is disposed at a predetermined water level, the medicinal-solution storing section storing the medicinal solution to a water level higher than the predetermined water level;
a liquid discharge section configured to discharge the medicinal solution from the medicinal-solution storing section;
a liquid supply section configured to supply liquid to the permeation membrane of the concentration meter; and
a control section connected to the liquid discharge section and the liquid supply section, the control section driving the liquid supply section to wet the permeation membrane when the medicinal solution in the medicinal-solution storing section is lower than the predetermined water level.

2. The endoscope reprocessor according to claim 1, wherein the liquid supply section includes a nozzle in which an opening is disposed to be directed to the permeation membrane and discharges the liquid from the nozzle toward the permeation membrane.

3. The endoscope reprocessor according to claim 1, wherein the liquid supply section includes a nozzle in which an opening is disposed higher than the permeation membrane in a gravity direction and discharges the liquid from the nozzle toward the permeation membrane.

4. The endoscope reprocessor according to claim 1, wherein the control section intermittently drives the liquid supply section.

5. The endoscope reprocessor according to claim 1, further comprising a detecting section configured to detect a dried state of the permeation membrane, wherein
the control section drives the liquid supply section based on a detection result of the detecting section.
